# EUROPEAN PATENT SPECIFICATION

(11) **EP 1 664 064 B1**
(45) Date of publication and mention of the grant of the patent: **10.12.2008**
(21) Application number: 04770462.2
(22) Date of filing: 02.09.2004
(51) Int. Cl.: C07F 5/02, A61K 31/69, A61P 31/00

(54) **OXAZABOROLIDINES AS BACTERIA EFFECTORS**
OXAZABOROLIDINE ALS BAKTERIELLE EFFEKTOREN
OXAZABOROLIDINES CONSTITUANT DES EFFECTEURS BACTERIENS

(30) Priority: 02.09.2003 US 499005 P
(43) Date of publication of application: 07.06.2006
(73) Proprietor: YISSUM RESEARCH DEVELOPMENT COMPANY OF THE HEBREW UNIVERSITY OF JERUSALEM, Givat Ram, Jerusalem 91390 (IL)
(72) Inventor: SREBNIK, Morris, 90805 Mevasseret Zion (IL); JABBOUR, Adel, 17510 Nazareth Elith (IL); MOUSSAIEFF, Arik, 96584 Jerusalem (IL); DEMBITSKY, Valery, 98300 Maale Adumim (IL); BRONSHTEYN, Moshe, 90100 Kiryat Arba (IL); STEINBERG, Doron, 97234 Jerusalem (IL)
(74) Representative: Grünecker, Kinkeldey, Stockmair & Schwanhäusser Anwaltssozietät
(86) International application number: PCT/IL2004/000791
(87) International publication number: WO 2005/021559

(56) References cited:
- WO-A-00/14236
- WO-A-03/018029
- JABBOUR, ADEL ET AL: "Synthesis and Evaluation of Oxazaborolidines for Antibacterial Activity against Streptococcus mutans" JOURNAL OF MEDICINAL CHEMISTRY , 47(10), 2409-2410 CODEN: JMCMAR; ISSN: 0022-2623, 6 May 2004 (2004-05-06), XP002314023
- CONTRERAS, ROSALINDA ET AL: "The nitrogen-boron coordination in hindered cyclic thexylboronic esters derived from diethanolamines" JOURNAL OF ORGANOMETALLIC CHEMISTRY , 246(3), 213-17 CODEN: JORCAI; ISSN: 0022-328X, 1983, XP002314024
- KUZNETSOV, V. V. ET AL: "Stereochemical peculiarities of transesterification of (N-B)-perhydro-2-isopropyl-1,3-dioxa-6-aza -2-boracine by 2-alkyl-1,3-butanediols" KHIMIYA GETEROTSIKLICHESKIKH SOEDINENII , (7), 994-6 CODEN: KGSSAQ; ISSN: 0453-8234, 1989, XP009042765
- SHITOV, O. P. ET AL: "Reaction of diethylborohalides with sodium salts of nitroalkanes" ZHURNAL OBSHCHEI KHIMII , 43(6), 1266-71 CODEN: ZOKHA4; ISSN: 0044-460X, 1973, XP009042766

## Description

The present invention concerns oxazaborolidines and more particularly, oxazaborolidines having biological effects, more particularly on bacteria.

Most bacteria in nature do not exist as isolated entities but assemble as communities attached to surfaces (Biofilms) which provides a sheltered microenvironment for immobilized bacteria. The biofilm is composed of cells, bacteria and cell-free organelles, all embedded in an extracellular matrix (polysaccharides).

Biofilms are diverse microbial communities embedded in a matrix of bacterial and host-origin constituents. The formation and maturation of the biofilm follows a series of dynamic biological events. The mechanisms of biofilm formation are complex. The preliminary stage is the formation of an acquired pellicle (conditioning film) comprised of cell-free host constituents, early bacterial colonizers adhere to the pellicle. This is followed by adhesion of late bacterial colonizers and co-adhesion. Next, bacteria propagate within the biofilm, after which a steady state is achieved with the surrounding environment.

Several adhesion avenues are associated with the formation of a biofilm. The most predominant type of adhesion involved polysaccharides. For example the dental biofilm: the sucrose-dependent adhesion, mediated by synthesis of polysaccharides by bacterial extracellular enzymes such as glucosyltransferase (GTF) and fructosyltransferase (FTF) is the pivotal avenue of bacterial adhesion. GTF catalyzes the synthesis of glucan type polysaccharides from sucrose substrate by polymerizing the glucosyl moiety of the sucrose into glucans which are considered as one of the major mediators in bacterial adhesion to dental surfaces. FTF is another extracellular enzyme found in the biofilm. It synthesizes fructan polymers from sucrose. FTF originates from oral bacteria such as *S. salivarius* or Actinomyces spp. and plays a role in carbohydrate metabolism, carbohydrate reservoir and as binding sites for bacteria.

Lately the scientific community has regarded the immobilized bacteria as biofilms which bear cell-cell communication (quorum sensing) (E. Peter Greenberg, Nature, 2003, 424, p. 134). Bacteria may communicate via several inducers (B.L. Bassler, Cell, 2003, 109, 421-424). Auto inducer-2 (AI-2), have been found in gram positive oral and gram negative oral bacteria such as *S. mutans* and *P. gingivitis* (P.E. Kolenbrander et al., Annu. Rev. Microbiol., 2000, 54, 413-437). This inducer is responsible for turning on vast metabolic and catabolic pathways. Only recently an unexpected finding has shown that AI-2 contains a boron component (X. Chen et al., Nature, 2002, 415, 545-549). The following cascade was suggested for the role of AI-2 on quorum sensing: the enzyme LuxS is responsible for the cleavage of S-ribosylhomocysteine to produce 4,5-dihydroxy-2,3-pentanedione (DPD); the Cyclic form of DPD (pro-AI-2) reacts with environmental boric acid to form the cyclic borate diester which serves as the core for the AI-2.

Quorum sensing induction depends on the presence of functional LuxP and LuxQ proteins. Chen *et al*., (X. Chen et al., Nature, 2002, 415, 545-549) concluded that availability of borate is a limiting factor in AI-2 formation. Such serendipitous findings (S. J. Coulthurst et al., Trends in Biochemical Sciences, 2002, 27, 217-219) of the structure for AI-2 and the role of boronic acid needs to be further established.

AI-2 has been proposed to serve as a 'universal' bacterial quorum-sensing signal containing boron for inter bacteria community communication (X. Chen et al., Nature, 2002, 415, 545-549). The finding that a boronic molecule plays an important role on quorum sensing is a surprising new data which needs to be elucidate.

US Patent 6,737,415 and WO 03/018029 refer to oxaanion compounds containing boron Phosphate, Sulfate which are used to influence the development and maintenance of biofilms, *inter alias* by effecting quorum-sensing.

WO 00/32152 discloses a compound known as auto inducer-2 (AI-2), which controls quorum sensing by binding to LuxP, a periplasmic binding protein from *Vibrio harveyi*. Crystallographic work on a luxP-AI-2 co-crystal, which resulted fro LuxP expressed by recombinant *Escherichia coli* in the presence of biologically-produced AI-2, yielded the a structure in which AI-2 (in the hydrated, gem-diol form of the keto group) binds at least in part to LuxP through the intermediacy of another species.

This intermediary species was initially believed to be a carbonate but subsequently recognized to be a borate moiety, with the borate possibly arising from adventitious borate derived from borosilicate glass used in the experimental work.

Recently AI-2 was found to contain a furanosyl borate diester (X. Chen et al., Nature, 2002, 415, 545-549).

Oxazaborolidines are five-membered heterocycles containing a B-N bond and an O atom having a B-N bond, obtained from a reaction between amino alcohol and boronic acids,. Oxazaborilidines are used in synthetic reactions, mainly in asymmetric organic synthesis, particularly in enantioselective reductions of ketons, imines, and oxime ethers, asymmetric Diels-Alder reactions, alcohol condensation and atroposelective reactions of lactones.

However, the use of boron-containing compounds in general and oxazaborolidines for medicinal purposes is rare.

Some success in the use of boron-containing compounds has been achieved in boron neutron capture therapy (BNCT). Very recently an α-amido boronic acid, Velcade, which serves as a a proteosome inhibitor, has been approved as an antineoplastic agent. Compounds containing B-N bonds have been shown to possess biological activity. Carboxyboranes have shown anticancer, hypolipidemic, and antifungal activity. Diazaborines have been shown to be active against malaria.

The present invention is based on the finding that several oxazaborolidines were found to have physiological/metabolic and enzymatic effects on bacteria. The oxazaborilides of the invention were found to modulate (increase or decrease) bacteria adhesion to the substrate; have anti-enzymatic bacterial activity; act as antibacterial agents; and influence quorum sensing between bacteria, which leads to decrease in communication between bacteria thus damaging the integrity of the bacterial biofilm. This damage makes the biofilm more vulnerable to harmful effects of the environment (erosion, effects of the immune system) as well as more vulnerable to other anti-bacterial agents.

The finding of the present invention paves the way to the production of novel compounds to be used as bacterial-modulating agents. The compounds of the invention can attack the bacteria through effecting various pathways, either one or several pathways simultaneously, such as by prevention of adhesion, by inhibition of bacterial enzymatic activity, by killing the bacteria, and by influencing the quorum sensing leading to biofilm disruption.

With the growing concern of bacterial resistance to traditional antibiotics, the development of any new compounds which can have antibacterial activity, or anti biofilm effect is always important.

Furthermore, the compounds of the present invention were found to be analogs (agonists or antagonists) of quorum sensing signals, and by this offer, another unique way of effecting bacteria, by disrupting bacteria-bacteria communication, and possibly also bacteria-host communication thereby mainly effecting film formation of bacteria in a non-violent manner. Such an anti-bacterial activity, may be more environmentally friendly as well as medicinally safe, as a selective pressure (resulting in the rise of resistance of bacteria) expected after treatment with the compound of the invention, should be minimal as compared to traditional antibacterial treatment which tend to select very rapidly for resistant bacteria which are now harder to effect..

The mechanism by which the oxazaborolidines effect bacteria is highly specific.e.g. its action is on quorum sensing is highly specific and is correlated to its structure.

The present invention is further based on the finding of several novel compounds of oxazaborolidines which are novel *per se.*

Thus, by one aspect, the present invention concerns a composition for modulation at least one bacteria-related parameter comprising a compound of formula I including a pharmaceutically acceptable salt, solvate, hydrate, isomers, and stereoisomers thereof: 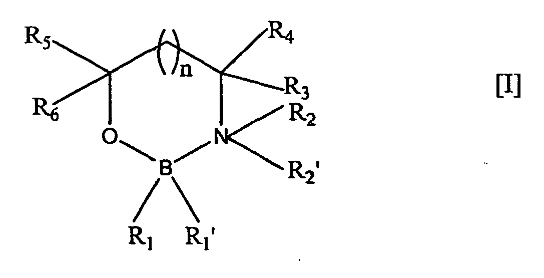 wherein,
n=0,1,2,3;
R₁ is selected from hydrogen, C₁-C₈alkyl, C₂-C₈ alkenyl, aryl, and C₃-C₇cycloalkyl;
R₂ is selected from hydrogen, C₁-C₈alkyl, C₂-C₈ alkenyl, aryl, C₃-C₇cycloalkyl, -(C=O)R, and -S(=O)₂R, where R is selected from C₁-C₈alkyl, aryl, and C₃-C₇cycloalkyl;
R₃ and R₄ are each independently selected from hydrogen, C₁-C₈alkyl, C₂-C₈ alkenyl, aryl, and C₃-C₇cycloalkyl;
R₅ and R₆ are each independently selected from hydrogen, C₁-C₈alkyl, C₂-C₈ alkenyl, aryl, and C₃-C₇cycloalkyl;
or one of R₃ and R₄ together with one of R₅ and R₆ form a substituted or unsubstituted aromatic ring, a substituted or unsubstituted cycloalkyl ring, or a substituted or unsubstituted heterocyclic ring, fused to the oxazaborolidine ring ;
R₁' is selected from null, hydrogen, hydroxyl, C₁-C₈alkyl, C₂-C₈ alkenyl and aryl;
R₂' is selected from null, hydrogen, C₁-C₈alkyl, C₂-C₈ alkenyl and aryl;
or R₁' together with R₂' are a group selected from -OR₁₀ -, -O-(C=O)R₁₀-, and - O-R₁₀(C=O)-, wherein R₁₀ is selected from a substituted or unsubstituted C₁-C₃alkyl, a substituted or unsubstituted aryl and a substituted or unsubstituted heteroaryl, thereby forming a ring fused to the oxazaborolidine ring.

The composition may be a pharmaceutical composition, an agricultural composition, a composition for industrial uses, or for general disinfection purposes. The composition preferably includes at least one compound of formula I and a carrier. The carrier may be a liquid carrier, a semi-solid carrier or a solid carrier.

The term *"modulating"* refers in the context of the present invention both to increasing the specific bacterial parameter as well as to decrease of parameter. Specific preferred modulatory effects will be discussed hereinafter in connection with each parameter.

The term *"bacteria-related parameter"* - refers in particular to one of the following:
1. Adhesion of the bacteria to its substrate. In this context, the term *"modulation "* can refer both to increase of the adhesion of the bacteria to the substrate, as well as to decrease of the adhesion to the substrate. The compounds of the invention can be grouped (by structure) to those that increase adhesion and those that decrease adhesion as will be explained herein bellow.
2. Decrease in enzymatic activity of the enzymes of the bacteria - in particular decrease in activity of the following enzymes, fructosyltransferase (FTF) and Glucosyltransferase (GTF) Preferably the compounds of the invention decrease the enzymatic activity of cell free enzymes (immobilized or in solution)
3. Viability of the bacteria - as determined by bacterial counts. Preferably in this case the modulation is decreased in the viability - i.e. affecting the integrity of the membrane or a cytotoxic effect on the bacteria.
4. Effect on quorum sensing - i.e. effect on bacteria-bacteria communication (and possibly bacteria-host communication) and here the modulation is preferred by influencing the quorum sensing (either decreasing or increasing the quorum sensing.
5. Biofilm formation by the bacteria - which is a combination of several activities concerning both the adhesion to the substrate, the quorum sensing, and affecting bacteria which participate in the biofilm formation . Typically, the modulation is disruption of the biofilm formation process resulting in a weaker film, prevention of formation of biofilm altogether; or damage to existing biofilm previously formed. A weaker biofilm is more vulnerable to subsequent attack by the environment (toxic substances, pH , erosion, the immune system) as well as to an attack by antibacterial agents

The term *"bacteria"* in the context of the present invention refers both to gram positive and gram negative bacteria, as both are known to be effective by quorum sensing. This term refers both to planktonic bacteria as well as to biofilm forming bacteria as some of the effects of the compounds of the invention are not related to quorum sensing (such as the cytotoxic effect and the anti-enzymatic effect ) and thus would also affect planktonic bacteria.

However, in accordance with a preferred embodiment of the present invention the bacteria are biofilm forming bacteria. The bacterial effected by the invention are from the group consisting of gram negative or gram positive bacteria, mycobacteria, as streptococci, staphylococci, Actinomyces, lactobacillus, as; Vibrio harveyi, Vibrio cholerae, Vibrio parahaeniolyticus, Vibrio alginolyticus, Pseudomonas phosphoreu77i, Yérsinia enterocolitica, Escherichia coli, Salmonella typhimuriuni, Haemophilus influenzae, Belicobacter pylori, Bacillus subtifis, Borrelia burgfUórferi, Néisseria meningitidis, Néisseria gonorrhocae, Yersinia pestis, Canipylobacter jejuni, Deinococcus radiodurans, Mycobacterium tuberculosis, Enterococeus faecalis, Streptococcus pneumoniae, Streptococcus pyogenes and Staphylococcus aureus. Most preferably, oral bacteria such asprophyromonas gingivalis, mutans streptococoii, Actinubacillus actinomycetemcomitans, pseudomonas argenoza.

As used herein the term **"C₁-C₈alkyl"** refers to a saturated aliphatic hydrocarbon of 1 to 8 carbon atoms. The C₁-C₈alkyl may be a straight or a branched alkyl. The C₁-C₆alkyl group may be for example methyl, ethyl, propyl, isopropyl, butyl, isobutyl, tertiary butyl, sec-butyl, amyl, pentyl, isopentyl, hexyl etc.

Whenever a numerical range e.g. "1-8" is stated herein, it means that the group in this case the alkyl group, may contain 1 carbon atom, 2 carbon atoms, 3 carbon atoms, 4 carbon atoms, etc., up to and including 8 carbon atoms.

The term **"C₂-C₈ alkenyl"** as used herein refers to unsaturated groups of 2 to 8 carbon atoms which contain at least one carbon-carbon double bond and includes straight-chain, branched-chain, and cyclic groups, all of which can be optionally substituted..̅ Non limiting examples of alkenyl groups are ethenyl, 1-propenyl, 2-propenyl, 2-methyl-1-propenyl, 1-butenyl, 2-butenyl and the like.

The term **"aryl",** as used herein, means a substituted or unsubstituted aromatic carbocyclic ring which may be monocyclic or fused aromatic ring (i.e., rings which share an adjacent pair of atoms) of 6 to 12 carbon atoms. Non limiting examples of aryl groups are phenyl, naphthyl, and the like. Preferably the aryl is phenyl. The aryl can be substituted by one or more, for example, one, two, three, or four identical or different substituents.

The term **"C₃-C₇cycloalkyl"** as used herein, means a saturated carboxylic ring of 3 to 7 carbon atoms, including but not limited to cyclopropyl, cyclobutyl, cyclopentyl, cyclohexyl, cycloheptyl and the like.

The term **"substituted or unsubstituted aromatic ring fused to the oxazaborolidine ring"** as used herein refers to substituted or unsubstituted aromatic carboxyclic ring which may be monocyclic or fused aromatic ring group of 6 to 12 carbon atoms, preferably the aromatic ring is monocyclic for example a benzene ring.

The term **"heterocyclic ring fused to the oxazaborolidine ring"** as used herein refers to a ring having at least 1 heteroatom such as nitrogen, sulfur and oxygen, including 5 to 10 membered (preferably 5 or 6 membered) aromatic heterocyclic ring and 5 to 10 membered (preferably 5 or 6 membered) nonaromatic heterocyclic ring. The 5 to 10 membered ring maybe monocyclic or bicyclic ring.

The N atom of the heterocyclic ring may be further substituted by a - (C=O)R group wherein R may be for example a C₁-C₈alkyl, C₂-C₈alkenyl, C₃-C₇cycloalkyl or aryl.

Non-limiting examples of aromatic heterocyclic rings are pyrrole, pyridine, thiophene, or furan.

The term **"substituted or unsubstituted heteroaryl"** as used herein refers to a 5 to 10 membered (preferably 5 or 6 membered) aromatic heterocyclic ring having at least 1 heteroatom such as nitrogen, sulfur and oxygen. Non-limiting examples of aromatic heterocyclic rings are pyrrole, pyridine, thiophene, or furan. The 5 to 10 membered ring maybe monocyclic or bicyclic ring.

The substituted aromatic ring, substituted cycloalkyl ring, substituted heterocyclic ring, substituted C₁-C₃alkyl, substituted aryl, and substituted heteroaryl groups may be substituted with at least one substituent (which may be identical or different). Non limiting examples of substituents include hydroxy, halo, or a C₁-C₆ alkyl groups.

As used herein the term **"oxazaborolidine ring"** refers to the heterocyclic ring in formula I containing the -O-B-N- bond, where n=0,1,2,3 (i.e. the heterocyclic ring may be a 5-8 membered ring).

Preferably in compound of formula I, n=0.

When n=0, the compound of formula I is of formula II:

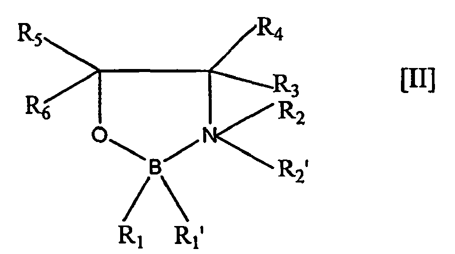

Where R₁, R₁', R₂, R₂', R₃, R₄, R₅, R₆ are as defined in formula I.

### In formula I or formula II:

Preferably the C₁-C₈alkyl is a C₁-C₄ alkyl. Most preferably the C₁-C₈alkyl is a methyl.

Preferably R₁' is null or hydroxyl, R₂ is null or hydrogen, and R₁ is C₁-C₈ alkyl. More preferably R₁ is butyl.

According to a preferred embodiment of the present invention, R₁' is null and R₁ is a butyl. More preferably R₁' is null, R₂' is null and R₁ is a butyl.

Preferably R₁ is C₁-C₈ alkyl and R₁' together with R₂ are a -OR₁₀ - group wherein R₁₀ is a substituted or unsubstituted C₁-C₃alkyl. More preferably the - OR₁₀- group is -O(CH₂)₂-.

Preferably R₁' is null or hydroxyl, R₂' is null or hydrogen, and R₁ is an aryl. Most preferably the aryl is phenyl.

Further according to a preferred embodiment of the present invention, R₁' is null and R₁ is an aryl.

Still further according to a preferred embodiment of the present invention, the aryl is phenyl.

Preferably R₁' is null, R₂' is null and R₁ is an aryl more preferably a phenyl. Preferably R₁' is hydroxyl, R₂' is hydrogen and R₁ is an aryl more preferably a phenyl.

Preferably R₁ is an aryl and R₁' together with R₂' are a -OR₁₀- group wherein R₁₀ is a substituted or unsubstituted C₁-C₃alkyl. More preferably -OR₁₀-group is -O(CH₂)₂-. Preferably the aryl of R₁ is phenyl.

Additionally according to a preferred embodiment of the present invention, R₁' together with R₂' are a -OR₁₀- group wherein R₁₀ is a substituted or unsubstituted C₁-C₃alkyl.

According to a preferred embodiment of the present invention, R₁₀ is - (CH₂)ₘ-, wherein m=1-3.

Moreover according to a more preferred embodiment of the present invention, -OR₁₀- group is -O(CH₂)₂-.

Further according to a preferred embodiment of the present invention, R₁' is hydroxyl and R₂' is hydrogen.

Still further according to a preferred embodiment of the present invention, R₁' and R₂' are null.

Moreover according to a preferred embodiment of the present invention when R₁' is hydroxyl, R₂' is hydrogen and vice versa, when R₂' is hydrogen R₁' is hydroxyl.

Additionally according to a preferred embodiment of the present invention when R₁' is null, R₂' is null and vice versa, when R₂' is null R₁' is null.

Preferably R₂' is null and R₂ is C₁-C₈alkyl. More preferably the C₁-C₈alkyl of R₂ is C₁-C₄alkyl. Most preferably C₁-C₈ alkyl of R₂ is a methyl.

Preferably R₂' is hydrogen, R₁' is hydroxyl and R₂ is C₁-C₈alkyl. Most preferably the C₁-C₈alkyl of R₂ is methyl.

Additionally according to a preferred embodiment of the present invention, the aromatic ring fused to the oxaborolidine ring is benzene. Examples of compounds wherein the aromatic ring is fused to the oxaborolidine ring are compounds (1), (2), (9) and (10), described in compound scheme I.

Moreover according to a preferred embodiment of the present invention, the cycloalkyl ring fused to the oxaborolidine ring is a C₃-C₇cycloalkyl.

Further according to a preferred embodiment of the present invention, the heterocyclic ring fused to the oxaborolidine ring is an aromatic heterocyclic ring selected from pyrrole, pyridine, thiophene, and furan.

Still further according to a preferred embodiment of the present invention, the nitrogen atom of said pyrrole is further substituted by -(C=O)R wherein R is selected from a C₁-C₈alkyl, C₂-C₈alkenyl, C₃-C₇cycloalkyl and aryl.

Non-limiting examples of compounds of formula I (or formula II) where one of R₃ and R₄ together with one of R₅ and R₆ form a substituted or unsubstituted aromatic ring, a substituted or unsubstituted heterocyclic ring, fused to the oxazaborolidine ring , are compounds (1)-(10) described below in compound scheme I. Similarly a substituted or unsubstituted cycloalkyl ring fused to the oxazaborolidine ring may also be formed.

In these compounds (compounds (1)-(10) of compound scheme I) R₁' together with R₂' are a group selected from -OR₁₀ -, -O-(C=O)R₁₀-, and -OR₁₀(C=O)-, wherein R₁₀ is selected from a substituted or unsubstituted C₁-C₃alkyl, a substituted or unsubstituted aryl and a substituted or unsubstituted heteroaryl, thereby forming a ring fused to the oxazaborolidine ring.

As used herein the term **"forming a ring fused to the oxazaborolidine ring"** refers for example to a monocyclic or bicyclic ring, depending on the R₁₀ group. For example when the R₁₀ is a C₁-C₃ alkyl the ring formed is monocyclic. When the R₁₀ is an aryl such as phenyl, or a heteroaryl such as for example pyrrole, pyridine, thiophene, or furan the ring formed fused to the oxazaborolidine is a bicyclic ring.

Moreover according to a preferred embodiment of the present invention, one of R₃ and R₄ together with one of R₅ and R₆ are -(CH₂)X(CH₂)ₚ-, wherein p=1,2,3, X=O, S, N-R', N(C=O)R", wherein R' is selected from hydrogen, C₁-C₈alkyl, C₂-C₈alkenyl, C₃-C₇cycloalkyl, and aryl, R" is selected from C₁-C₈alkyl, C₂-C₈alkenyl, C₃-C₇cycloalkyl, and aryl, thereby forming a heterocyclic ring fused to the oxazaborolidine ring.

According to a preferred embodiment of the present invention, n=0, R₁ is selected from C₁-C₈alkyl and aryl; R₂ is selected from hydrogen and C₁-C₈alkyl; R₃ and R₄ are each independently selected from hydrogen, C₁-C₈alkyl and aryl; R₅ and R₆ are each independently selected from hydrogen, C₁-C₈ alkyl and aryl; R₁' is selected from null and hydroxyl; R₂' is selected from null and hydrogen; or R₁' together with R₂' are a -OR₁₀- group, wherein R₁₀ is C₁-C₃ alkyl, thereby forming a heterocyclic ring fused to the oxazaborolidine ring.

Additionally according to a more preferred embodiment of the present invention, n=0, R₁ is selected from C₁-C₈alkyl and aryl; R₂ is selected from hydrogen and C₁-C₈alkyl; R₃ and R₄ are each independently selected from hydrogen, C₁-C₈alkyl and aryl; R₅ and R₆ are each independently selected from hydrogen, C₁-C₈ alkyl and aryl; R₁' is selected from null and hydroxyl; and R₂' is selected from null and hydrogen.
Preferably R₁ is C₁-C₈ alkyl. More preferably R₁ is C₁-C₄ alkyl and most preferably R₁ is butyl.
Preferably R₁' is null and R₁ is a butyl.
Preferably the aryl is a phenyl.
Preferably R₁ is an aryl. More preferably the aryl is phenyl.
Preferably R₁' is null and R₁ is an aryl, more preferably the aryl is phenyl.

Preferably R₂ is C₁-C₈alkyl. More preferably the C₁-C₈alkyl of R₂ is C₁-C₄alkyl. Most preferably the C₁-C₈alkyl of R₂ is methyl.

According to a preferred embodiment of the present invention, R₁' and R₂' are null.

Further according to a preferred embodiment of the present invention, R₁' is hydroxyl and R₂' is hydrogen.

Still further according to a preferred embodiment of the present invention, C₁-C₈alkyl of R₃ and R₄ is methyl.

Additionally according to a preferred embodiment of the present invention, one of R₅ and R₆ is an aryl and the other is hydrogen.

Additionally according to a preferred embodiment of the present invention, the aryl is phenyl.

According to one preferred embodiment of the present invention, R₁' and R₂' are null.

According to another preferred embodiment of the present invention, R₁' is hydroxyl and R₂' is hydrogen.

Additionally according to a more preferred embodiment of the present invention, the compound of formula I is selected from:
3,4-dimethyl-2,5-diphenyl-1,3,2-oxazaborolidine;
4,4-dimethyl-2-phenyl-1,3,2-oxazaborolidine;
2-butyl-4,4-dimethyl-1,3,2-oxazaborolidine;
4-methyl-2,5-diphenyl-1,3,2-oxazaborolidine;
2-butyl-3,4-dimethyl-5-phenyl-1,3,2-oxazaborolidine;
2-butyl-4-methyl-5-phenyl-1,3,2-oxazaborolidine;
B-hydroxy-3,4-dimethyl-2,5-diphenyl-1,3,2-oxazaborolidine;
B-hydroxy-4,4-dimethyl-2-phenyl-1,3,2-oxazaborolidine;
B-hydroxy-2-butyl-4,4-dimethyl-1,3,2-oxazaborolidine;
B-hydroxy-4-methyl-2,5-diphenyl-1,3,2-oxazaborolidine;
B-hydroxy-2-butyl-3,4-dimethyl-5-phenyl-1,3,2-oxazaborolidine; and
B-hydroxy-2-butyl-4-methyl-5-phenyl-1,3,2-oxazaborolidine.

The preferred compounds are presented in the table I and II below:

**Table I: Compounds BNO1-BNO6**

| **Compound Number** | **Structure** | **Chemical Name** |
|---|---|---|
| BNO1 | 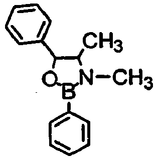 | 3,4-dimethyl-2,5-diphenyl-1,3,2-oxazaborolidine |
| BN02 | 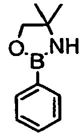 | 4,4-dimethyl-2-phenyl-1,3,2-oxazaborolidine |
| BN03 | 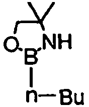 | 2-butyl-4,4-dimethyl-1,3,2-oxazaborolidine |
| BN04 | 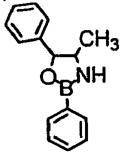 | 4-methyl-2,5-diphenyl-1,3,2-oxazaborolidine |
| BNO5 | 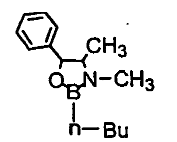 | 2-butyl-3,4-dimethyl-5-phenyl-1,3,2-oxazaborolidine |
| BN06 | 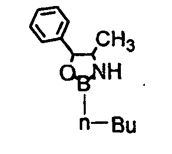 | 2-butyl-4-methyl-5-phenyl-1,3,2-oxazaborolidine |
| BN07 | 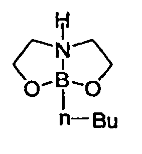 | [(2-)-N,O,O'[2,2'-Iminobis[ethanolato]]]-2-n-butylboron |
| | | or |
| | | 2-n-butyl-tetrahydro-[1,3,2]oxazaborolo[2,3-b][1,3,2]oxazaborole |
| BNO8 | 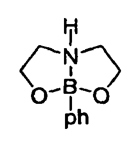 | [(2-)-N,O,O'[2,2'-Iminobis[ethanolato]]]-2-phenylboron |
| | | or |
| | | 2phenyl--tetrahydro-[1,3,2]oxazaborolo[2,3-b][1,3,2]oxazaborole |

| | | |
|---|---|---|
| * In the present invention BNOO1 refers to BN07; BNOO2 refers to BNO8. | | |

**Table II: Hydrates of compounds BNO1-BNO6**

| **Compound number** | **Structure Chemical Name** | |
|---|---|---|
| **BNO1 hydrate** | 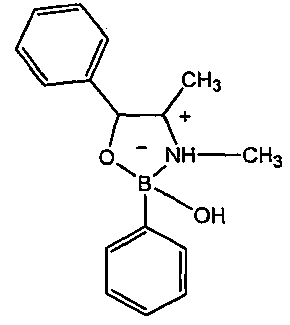 | B-hydroxy-3,4-dimethyl-2,5-diphenyl-1,3,2-oxazaborolidine |
| **BN02 hydrate** | 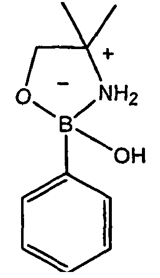 | B-hydroxy-4,4-dimethyl-2-phenyl-1,3,2-oxazaborolidine |
| **BN03 hydrate** | 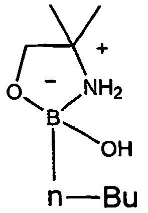 | B-hydroxy-2-butyl-4,4-dimethyl-1,3,2-oxazaborolidine |
| **BN04 hydrate** | 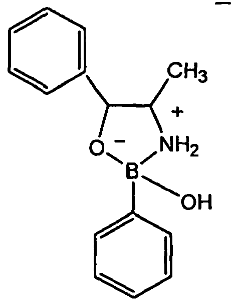 | B-hydroxy-4-methyl-2,5-diphenyl-1,3,2-oxazaborolidine |
| **BNO5 hydrate** | 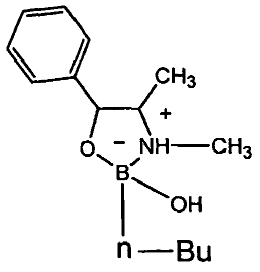 | B-hydroxy-2-butyl-3,4-dimethyl-5-phenyl-1,3,2-oxazaborolidine |
| **BN06 hydrate** | 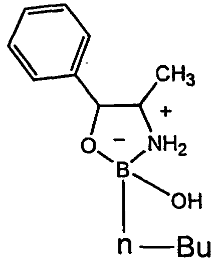 | B-hydroxy-2-butyl-4-methyl-5-phenyl-1,3,2-oxazaborolidine |

| | | |
|---|---|---|
| * In the above compounds n-Bu is n-butyl. | | |

Additional examples of compounds which can be used in the present invention are presented in the following compounds scheme:

### Compound scheme I:

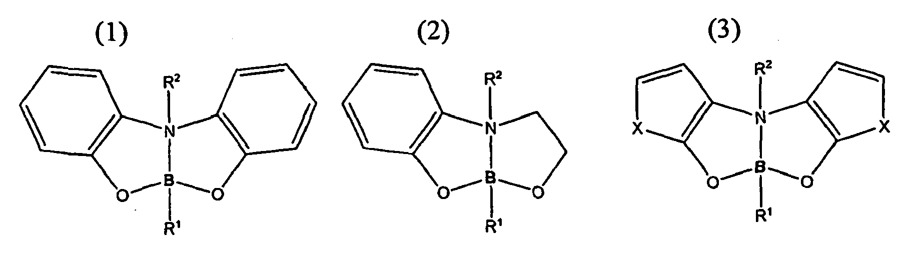

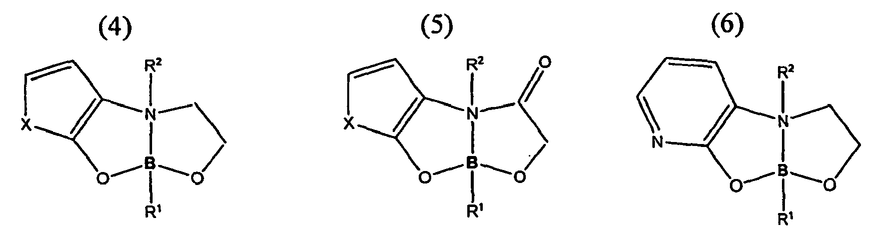

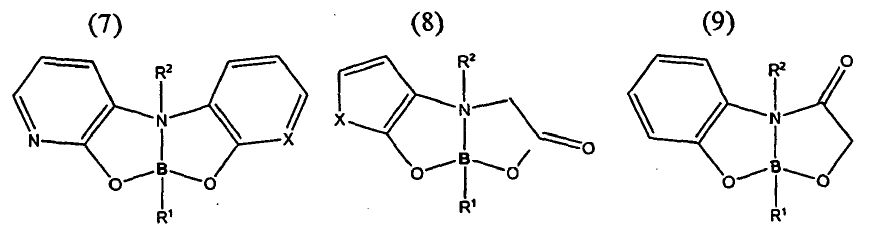

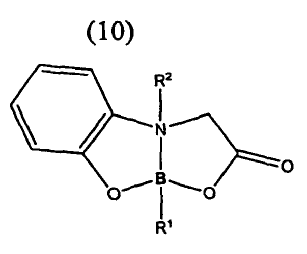

In the above compounds (1-10), R₁ and R₂ may be as defined in formula I. R₁ is selected from hydrogen, C₁-C₈alkyl, C₂-C₈ alkenyl, aryl, and C₃-C₇cycloalkyl;

R₂ is selected from hydrogen, C₁-C₈alkyl, C₂-C₈ alkenyl, aryl, C₃-C₇cycloalkyl, -(C=O)R, and -S(=O)₂R, where R is selected from C₁-C₈alkyl, aryl, and C₃-C₇cycloalkyl.

In the above compounds, X is O, S, NH, or N(C=O)R, wherein R is selected from C₁-C₈alkyl, C₂-C₈alkenyl, C₃-C₇cycloalkyl or aryl.

The aromatic and heterocyclic rings of compounds 1-10 may be further substituted with at least one substituent (which may be identical or different).

The substituents my be for example hydroxy, halo, a C₁-C₆ alkyl group.

Moreover according to a preferred embodiment of the present invention, n=0, R₁ is selected from C₁-C₈ alkyl and aryl; R₂ is selected from hydrogen and C₁-C₈alkyl; R₃ and R₄ are each independently selected from hydrogen, C₁-C₈alkyl and aryl; R₅ and R₆ are each independently selected from hydrogen, C₁-C₈alkyl and aryl; and R₁' together with R₂' are a -OR₁₀- group, wherein R₁₀ is C₁-C₃ alkyl, thereby forming a heterocyclic ring fused to the oxazaborolidine ring.

The -OR₁₀- group, wherein R₁₀ is C₁-C₃ alkyl is of the structure -O(CH₂)ₘ-, wherein m=1-3.

Most preferably R₁₀ is ethyl. (Most preferably *m* in -O(CH₂)ₘ-, is 2).
Preferably R₁ is aryl and more preferably the aryl of R₁ is a phenyl.
Preferably the C₁-C₈alkyl is C₁-C₄ alkyl.
Preferably R₁ is C₁-C₈ alkyl, more preferably R₁ is C₁-C₄ alkyl. Most preferably the C₁-C₈alkyl of R₁ is a butyl.

Moreover according to a preferred embodiment of the present invention, R₂ is hydrogen.

Preferably R₂ is Cₗ-C₈ alkyl, more preferably C₁-C₄ alkyl, and most preferably the C₁-C₈alkyl of R₂ is methyl.

Moreover according to a preferred embodiment of the present invention, R₃, R₄, R₅ and R₆ are hydrogen.

Most preferably the compound is selected from:
[(2-)-N,O,O'[2,2'-Iminobis[ethanolato]]]-2-n-butylboron; and
[(2-)-N,O,O'[2,2'-Iminobis[ethanolato]]]-2-phenylboron.

Preferably the at least one bacteria-related parameter is selected from:
(a) adhesion of the bacteria to its substrate;
(b) enzymatic activity of the enzymes;
(c) viability of the bacteria;
(d) effect on quorum sensing;
(e) biofilm formation by the bacteria;
(f) a combination of two or more of (a)-(e).

Preferably the modulation is decrease of the bacteria-related parameter. Preferably the bacteria-related parameter is adhesion of the bacteria to its substrate and said modulation is increase.

Preferably the bacteria related parameter is viability of bacteria, the modulation is decrease and the compound is selected from 3,4-dimethyl-2,5-diphenyl-1,3,2-oxazaborolidine (BNO**1**); and
and 2-butyl-3;4-dimethyl-5-phenyl-1,3,2-oxazaborolidine (BNO**5**).

Preferably the bacteria related parameter is adhesion of the bacteria to its substrate, the modulation is decrease, and the compound is selected from 2-butyl-4,4-dimethyl-1,3,2-oxazaborolidine (BNO**3**), 2-butyl-3,4-dimethyl-5-phenyl-1,3,2-oxazaborolidine (BNO**5**), 2-butyl-4-methyl-5-phenyl-1,3,2-oxazaborolidine (BNO**6**) and [(2-)-N,O,O'[2,2'-Iminobis[ethanolato]]]-2-n-butylboron (BNO**7**).

Preferably the bacteria related parameter is adhesion of the bacteria to its substrate, the modulation is increase, and the compound is selected from 3,4-dimethyl-2,5-diphenyl-1,3,2-oxazaborolidine (BNO**1**), 4,4-dimethyl-2-phenyl-1,3,2-oxazaborolidine (BNO**2**), 4-methyl-2,5-diphenyl-1,3,2-oxazaborolidine (BN04), and [(2-)-N,O,O'[2,2'-Iminobis[ethanolato]]]-2-phenylboron (BN08).

Preferably the bacteria related parameter is effect on quorum sensing, the modulation is increase, and the compound is 3,4-dimethyl-2,5-diphenyl-1,3,2-oxazaborolidine (BNO**1**);

Preferably the bacteria related parameter is effect on quorum sensing, said modulation is decrease, and said compound is 4,4-dimethyl-2-phenyl-1,3,2-oxazaborolidine (BN02).

By another aspect the present invention concerns a compound of formula I including a pharmaceutically acceptable salt, solvate, hydrate, isomers, and stereoisomers thereof: 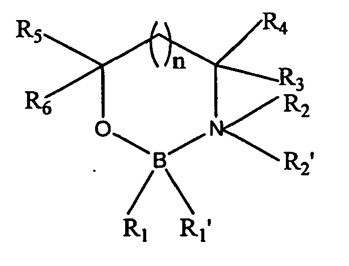 wherein,
n=0;
R₁ is selected from C₁-C₈ alkyl and aryl;
R₂ is selected from hydrogen and C₁-C₈alkyl;
R₃ and R₄ are each independently selected from hydrogen, C₁-C₈alkyl and aryl;
R₅ and R₆ each independently selected from hydrogen, C₁-C₈alkyl and aryl; and
R₁' together with R₂' are -OR₁₀- group, wherein R₁₀ is a C₁-C₃ alkyl, thereby forming a heterocyclic ring fused to the oxazaborolidine ring
with the proviso that the following compounds are excluded wherein
R₁ is C₂-C₄alkyl group or C₆alkyl group, R₁' together with R₂' are -OR₁₀- group,
wherein R₁₀ is C₂alkyl group, and R₂ to R₆ represent a hydrogen atom; and
R₁ is C₆alkyl group, R₁' together with R₂' are -OR₁₀- group, wherein R₁₀ is C₂alkyl group, and R₂ is a C₁alkyl group.

The -OR₁₀- group, wherein R₁₀ is C₁-C₃ alkyl is of the structure -O(CH₂)ₘ-, wherein m=1-3.

Most preferably R₁₀ is ethyl. (Most preferably *m* in -O(CH₂)ₘ-, is 2).
Preferably R₁ is aryl and more preferably the aryl of R₁ is a phenyl.

Preferably the C₁-C₈alkyl is C₁-C₄ alkyl.

Preferably R₁ is C₁-C₈ alkyl, more preferably R₁ is C₁-C₄ alkyl. Most preferably the C₁-C₈alkyl of R₁ is a butyl.

Moreover according to a preferred embodiment of the present invention, R₂ is hydrogen.

Preferably R₂ is C₁-C₈ alkyl, more preferably C₁-C₄ alkyl and most preferably the C₁-C₈alkyl of R₂ is methyl.

Moreover according to a preferred embodiment of the present invention, R₃, R₄, R₅ and R₆ are hydrogen.

Most preferably the compound is selected from:
[(2-)-N,O,O'[2,2'-Iminobis[ethanolato]]]-2-phenylboron.

By a preferred aspect the present invention concerns a composition for decreasing bacterial growth comprising a compound of Formula (I) as defined in the present invention.

The term "*decreasing bacterial growth*" refers to one of the following; decrease in the number of viable bacterial of at least one species; decrease in the rate of growth of the number of viable bacteria (although the number may increase); elimination of all viable bacteria; prevention of the formation and accumulation of bacteria on a specific target.

The present invention further concerns a composition for increasing the susceptibility of bacteria to the cytotoxic effects of other antibacterial agents comprising the compound of formula (I) as defined in the present invention..

The term "*increasing susceptibility to cytotoxic effects of other antibacterial agents*" refers to the fact that in the presence of the compound of the invention other anti-bacterial agents (antibiotics) have to be administered in smaller amounts as compared to control to obtain the same level of cytotoxic effect.

The compounds of the present invention may have several non medicinal utilities. For example the compounds of the invention may provide environmentally friendly antibacterial agents, such as for use in aquariums, in industrial applications using bacteria, industrial applications using bioreactors, cases where it is desired to prevent film forming bacteria such as inside pipes leading fluids. Another non-medicinal application is their use as pesticides to prevent film forming bacteria on crops. The compounds of the present invention can also be used to decrease bacteria growth on devices and compound used in therapeutic applications such on implants; (orthopedic, dental) artifical organs, tubes, catethers, zondas, feeding tubes, infusion tubes, dialysis instruments etc.

In addition, the invention can be used in biotechnology industries in which immobilization of bacteria physiology, communication and viability of bacteria is of major concern.

In most cases, the compounds of the invention would serve as antibacterial agents by affecting viability and/or adhesion to substrate and/or bacterial enzymatic activity and/or quorum sensing and/or biofilm maintenance, integrity or formation, or by a combination of the above.

However, it has been surprisingly found that some oxazaborolidines of the present invention improve adhesion of the bacteria to their substrates.

The property of improving adhesion, and biofilm integrity may help in bio production processes ,in maintaining a stable population of cells with decreased erosion ,as substrates, fluids and gasses pass through a bio reactor without causing erosion. At times it is also desirable to enhance biofilm integrity or biofilm production also in a live host for example of germs of non pathogenic effects - which counter action a pathological situation as in stomach, vagina, in agriculture, or bacteria replacement therapy.

The most interesting activity of the compounds of the invention is as pharmaceutical compositions.

Thus, by another aspect of the present invention, the present invention concerns a pharmaceutical composition comprising a pharmaceutically acceptable carrier, and as an active ingredient a compound having formula I as defined in the present invention.

The pharmaceutical composition of the present invention should preferably be for the treatment, prevention, or amelioration of bacterial infection.

Preferably the pharmaceutical composition is for increasing the susceptibility of bacteria to the cytotoxic effect of other antibacterial compounds.

Preferably the pharmaceutical composition further comprising at least one other antibacterial agent (compound).

The present invention further concerns the use of the compounds of formula I as defined in the present invention for the preparation of a pharmaceutical preparation for decreasing bacterial growth.

The use is preferably for the preparation of an anti-bacterial pharmaceutical preparation.

Preferably the bacteria are biofilm forming bacteria as described above.

The methods (both for treatment of bacterial growth and for increased susceptibility) may be used in connection with bacterial infections in a living host (mammal, poultry, fish, insect (bees)) and in that case the "*contact*" is either application of the compounds of the invention to an external part of the host (skin, teeth, fur, feathers, ears, eyes, mucosal tissue) or by systemic administration to the host in one of the manners that will be indicated herein bellow.

The term "*effective amount*" in connection with the modulation aspect reefers to an amount which changes (increases or decreases the bacterial parameters in a statistically significant manner as compared to control.

The term "*effective amount*" in connection with the treatment, decrease or prevention method, of bacterial growth ,refers to an amount that can prevent bacterial infection after contact of the host with an infective source, decrease the number of viable bacteria in the host, or eliminate the bacteria altogether, in a statistically significant manner as compared to control.

The term "*effective amount*" in connection with the susceptibility increase to antibiotics, refers to an amount which is capable to decreasing the amount of the other antibiotics, in a statistically significant manner, so that the other compound can be administered in smaller amounts to obtain the same effect of an administration of the other anti-bacterial agent (antibiotics) alone.

The mechanism of effecting bacterial infection is as defined above under the term "*bacterial-related parameter*".

The bacteria in the mode of treatment by the compound of the invention is again as described above in connection with the term "*bacteria*".

### Preparation of the compounds:

The compounds of the present invention can be prepared according to the methods described in: R. Koster. Organoboron Compounds I, in Houben-Weyl: Handbook of Organic Chemistry, 4th Ed.;G. Thieme Verlag: 1982, Vol 13/3a, p. 157, incorporated herein by reference in its entirety, or a modification thereof which will be apparent to those skilled in the art.

The oxazaborolidines of the present invention (such as compounds BNO**1-6)** may be synthesized by the reaction of an amino alcohol using the (-)-ephedrine, (-)- norpseudoephedrine, or a diol amine and an appropriate boronic acid, with the azeotropic removal of water as described in reaction scheme I.

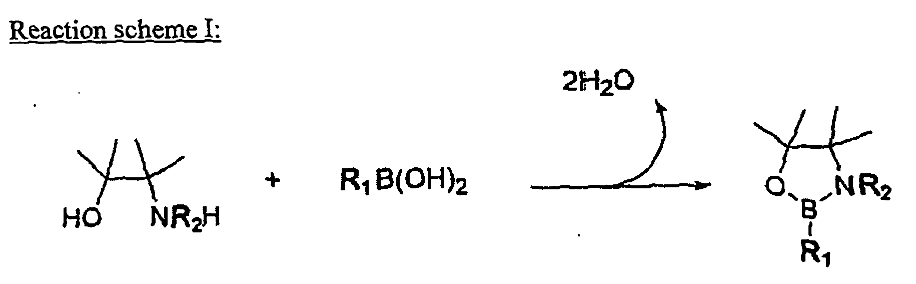

R₁ and R₂ maybe as described in the present invention.

Preferably the reaction mixture is refluxed for preferably 2-3 hours, in an organic solvent such as tetrahydrofuran (THF) or tuluene, preferably toluene and the water is removed.

The compound is isolated by evaporating the organic solvent preferably toluene. Then the residue is vacuum distilled to obtain the desired compound.

The compounds of the present invention (such as BNO**7** and BNO**8**) may be prepared by the following process:
an amino alcohol preferably a diol amine and most preferably diethanolamine is reacted with an appropriate boronic acid preferably C₁-C₈alkyl boronic acid (more preferably butyl boronic acid) or aryl boronic acid (more preferably phenyl boronic acid) with the azeotropic removal of water as described in reaction scheme I.

In step (a) the amino alcohol is reacted with an appropriate boronic acid in an organic solvent preferably a mixture of ether and dichloromethane (other solvents or mixtures of solvents may alternatively be used depending on the solubility of the boronic acid and the aminoalcohol).

In step (b) the resulting reaction solution (preferably a heterogeneous solution) is stirred preferably for 1-2 hours and more preferably for 2 hours, preferably under inert conditions and more preferably under argon.

The process may further comprise isolating the obtained compounds by the following steps: triturating the obtained solid with an organic solvent such as ethylether/hexanes mixtures or dichloromethane, preferably dichloromethane; filtering and washing with an organic solvent such as hexane or dichloromethane preferably dichloromethane; concentrating the filtrate preferably under reduced pressure to obtain a solid ;

Preferably the process further comprises the steps of recrystallization the obtained solid by the following steps:
dissolving the obtained solid in an organic solvent more preferably hot organic solvent and most preferably hot dichloromethane; adding another organic solvent such as hexanes, petroleum ether, or ether preferably ether to induce recrystallization of the solid; cooling the mixture to a temperature of about 25°C, if necessary further cooling preferably to a temperature of 0°C;
preferably the recrystallization comprises the further steps of collecting the solid preferably by filtration and washing with an organic solvent preferably ether; and drying the product preferably under reduced pressure.

The hydrate compounds of the present invention may be obtained by dissolving the appropriate oxazaborolidine in water and stirring for about 2 hours.

### GENERAL DESCRIPTION OF THE INVENTION

One application of the compounds of the invention is in influencing the development or maintenance of biofilms which are communities of bacteria that grow attached to solid surfaces. Typically, bacteria within biofilms exhibit greater resistance to antibiotic treatments than those living freely as antibiotic compounds have a problem infiltrating into deeper layers of the biofilm. Such biofilm commonly lead to persistent and chronic infections refractory to treatment. The US Center for Disease Control estimates that 60% of bacterial infections involve such biofilm.

Industrially, biofilms contaminate and clog water lines, foul surfaces, and contribute to corrosion and decay. On the other hand, it is at times desired to improve biofilm formation, for example in bioproduction to maintain the integrity as substrate circulates through the bio reactor.

Quorum-sensing influences biofilm formation, and therefore ways of promoting or impeding quorum-sensing also provide ways of controlling biofilm formation, including biofilm growth. For example, compounds of structure I can be used to affect biofilms by either stimulating their formation or hindering it. Methods for promoting or impeding biofilm formation are preferably practiced by exposing the bacteria to the compound in an amount that affects biofilm formation. Particular amounts for a given application may be determined by routine experimentation in a manner generally known to those skilled in the art.

The final form of the composition of the present invention may be for example an emulsion, an aqueous solution, an oil, a semi-solid formulation (such as a cream, an ointment, a paste, or a gel), a lotion, a milk, a suspension, a powder, a capsule, a tablet, an aerosol, a spray, a lacquer, or an injection.

Reference to a particular compound herein is to be understood as a reference to the compound itself and any salts thereof, and vice versa. Compounds that possess an acidic or basic group may form pharmaceutically-acceptable salts with pharmaceutically-acceptable cations or anions. Examples of pharmaceutically-acceptable cations include ammonium, tetramethylammonium, alkali metal (e.g. sodium, lithium and potassium) and alkaline earth metal (e.g. calcium, barium and magnesium), aluminum, zinc, and bismuth cations, and protonated forms of basic amino acids, such as arginine, lysine, and organic amines such as ethanolamine, ethylenediamine, triethanoleamine, benzylphenethylamine, methylamine, dimethylamine, trimethylamine, diethylamine, piperidine, morpholine, tris-(2-hydroxyethyl)amine, and piperazine.

Examples of pharmaceutically-acceptable anions include those derived from inorganic acids such as hydrochloric, hydrobromic, hydriodic, sulfuric, and phosphoric acid, as well as organic acids such as p-toluenesulfonic, methanesulfonic, oxalic, p-bromo-phenylsulfonic, carbonic, succinic, citric, benzoic, and acetic acid, and related inorganic and organic acids. Such pharmaceutically-acceptable salts include sulfate, pyrosulfate, bisulfate, sulfite, bisulfite, phosphate, ammonium, monohydrogenphosphate, dihydrogenphosphate, meta-phosphate, pyrophosphate, chloride, bromide, iodide, acetate, propionate, decanoate, caprylate, acrylate, formate, isobutyrate, caprate, heptanoate, propiolate, oxalate, malonate, succinate, suberate, hippurate, butyne-1,4-dioate, hexane-1,6-diospate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, α-hydroxybutyrate, glycolate, maleate, tartrate, methanesulfonate, propanesulfonate, naphthalene-1-sulfonate, naphthalene-2-sulfonate, mandelate. It is understood that the above salts may form hydrates or exist in a substantially anhydrous form.

Where the bacterial infection is in a subject the compounds described herein may be administered directly to subjects, preferably humans, and/or may be administered in the form of pharmaceutical compositions comprising one or more of the compounds together with a pharmaceutically acceptable carrier, optionally together with another antibacterial agent. The application may either be external to by systhemic administration. A preferred mode of administration of the compound is oral. Another preferred route of administration is in the form of a dental composition (liquid, paste, salve) to be applied onto the teeth to eliminate the formation of bacteria of the species prophyromonas gingivalis, mutans streptococoii, or Actinubacillus actinomycetemcomitans known to contribute tooth decay. Oral compositions preferably include an inert diluent and/or an edible carrier. The compound can be enclosed in gelatin capsules or compressed into tablets. For the purpose of oral therapeutic administration, the compound can be incorporated with excipients and used in the form of tablets, troches, or capsules. Pharmaceutically compatible binding agents and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; and/or a flavoring agent such as peppermint, methyl salicylate, or orange flavoring. When the dosage unit form is a capsule, it can contain, in addition to material of the above type, a liquid carrier such as a fatty oil. In addition, dosage unit forms can contain various other materials which modify the physical form of the dosage unit, for example, coatings of sugar, shellac, or other enteric agents. The compound can be administered as a component of an elixir, suspension, syrup, wafer, chewing gum or the like. A syrup may contain, in addition to the compound, sucrose as a sweetening agent and preservatives, dyes and colorings and flavors.

The compound can also be mixed with other active materials that do not impair the desired action, or with materials that supplement the desired action, such as antibiotics that can be administered prior, after or during (co-administration) to the compounds of the invention using the same or a different mode of administration. It is known that penetration of antibiotics into "deeper" layer of the biofilm are problematic, the compounds of the invention which disrupt the integrity of the film can increase effectively of the antibiotic treatment, as the antibiotics penetrate into deeper layers of the biofilm.

Preferred antibiotics for this purpose include aminoglycosides such as tobramycin, glycopeptides such as vancomycin, beta lactams such as amoxicillin, quinolones such as ciprofloxicin, macrolides such as azithromycin, tetracyclines, sulfonamides, trimethoprim-sulfamethoxazole, or chloramphenicol. Solutions or suspensions used for parenteral, intradermal, subcutaneous, or topical application can include the following components: a sterile diluent such as water for injection, saline solution, fixed oils, polyethylene glycols, glycerine, propylene glycol or other synthetic solvents; antibacterial agents such as benzyl alcohol or methyl parabens; chelating agents such as ethylenediaminetetraacetic acid (EDTA); buffers such as acetates, citrates or phosphates and agents for the adjustment of tonicity such as sodium chloride or dextrose. The parental preparation can be enclosed in ampoules, disposable syringes or multiple dose vials made of glass or plastic. If administered intravenously, preferred carriers are physiological saline or phosphate buffered saline (PBS).

In one embodiment, the compound is prepared with carriers that protect it against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for the preparation of such formulations are known to those skilled in the art.

Pharmaceutical compositions are preferably administered to subjects, preferably humans, in an amount that is therapeutically effective to treat a bacterial infection. Therapeutically effective amounts can be determined by those skilled in the art by such methods as clinical trials. Dosage may be adjusted in individual cases as required to achieve the desired degree of target bacterial regulation. Sustained release dosages and infusions are specifically contemplated. Pharmaceutical compositions can be administered by any appropriate route for systemic, local or topical delivery, for example, orally, parenterally, intravenously, intradermally, subcutaneously, buccally, intranasally, by inhalation, vaginally, rectally or topically, in liquid or solid form . Methods of administering the compounds described herein may be by specific dose or by controlled release vehicles.

The pharmaceutical composition may be administered at once, or may be divided into a number of smaller doses to be administered at varying intervals of time. It is to be further understood that for any particular subject, specific dosage regimens should be adjusted over time according to the individual need and the professional judgment of the person administering or supervising the administration of the compound, and that the concentration ranges set forth herein are exemplary only and are not intended to limit the scope or practice of the claimed methods.

In a particular case, the therapeutically effective amount of a pharmaceutical composition to be used in the treatment of a bacterial infection will typically vary with the severity of the infection and the route by which the drug is administered. The dose, and perhaps the dose frequency, will also vary according to the age, body weight, and response of the individual patient. In general, the total daily dose range of the present compounds for a 70 kg person is from about 1 mg to about 2000 mg, in single or divided doses. Preferably, a daily dose range for a 70 kg person should be between about 5 mg and about 1500 mg, in single or divided doses. More preferably, a daily dose range for a 70 kg person should be between about 10 mg and about 1000 mg, in single or divided doses. In managing the patient, the therapy should be initiated at a lower dose, perhaps about 1 mg to about 200 mg for a 70 kg person, and increased up to about 1000 mg or higher depending on the patient's global response. It is further recommended that infants, children, patients over 65 years, and those with impaired renal or hepatic function, initially receive low doses, and that they be titrated based on individual response(s) and blood level(s). It may be necessary to use dosages outside these ranges in some cases as will be apparent to those skilled in the art. Further, it is noted that the clinician or treating physician will know how and when to interrupt, adjust or terminate therapy in conjunction with individual patient response. The terms *"therapeutic amount"* and *"therapeutically effective amount"* are encompassed by the above-described dosage amounts and dose frequency schedules.

The present invention further concerns the use of the compound of formula I, for the preparation of a pharmaceutical composition.

Preferably, the pharmaceutical composition is for the treatment bacterial infection, as per the definitions above.

### BRIEF DESCRIPTION OF THE DRAWINGS

In order to understand the invention and to see how it may be carried out in practice, some preferred embodiments will now be described, by way of non-limiting examples only, with reference to the accompanying drawings, in which:
**Fig. 1** shows the percentage of fructosyltransferase (FTF) activity, in the presence of varying concentrations of several compounds of the invention. Data presented as percent of activity compared to control with no tested compounds
**Fig. 2** shows dose-dependent adhesion of bacteria to the substrate in the presence of various compounds of the invention.
**Fig. 3** shows the bioluminescence induction of *Vibrio harveyi BB170* by varying concentration of the compound BNO-1 of the invention.
**Fig. 4** shows the bioluminescence in of *Vibrio harveyi BB170* with varying concentration of compounds BNO-2 of the invention. Bioluminescence was induced by the presence of AI-2. Data is presented as percentage of residual bioluminescence in the presence of BN02 to bioluminescence of bacteria with only AI-2.

### DETAILED DESCRIPTION OF THE INVENTION

### Example 1: Effect of the compounds of the invention on bacterial viability

### 1.1 Experimental

Sterile microculture dishes containing multiple wells were used to determine the minimal inhibitory concentration (MIC) of eight derivatives of oxazborolidines. Each well contained 120µl of brain heart infusion medium, 15µl of an overnight suspension of *S. mutans* ATCC 27351 adjusted to 1 OD₅₄₀ₙₘ and 15µl of the tested compound. Each compound was tested at five different concentrations between 0-50 mM. Each experiment was repeated twice. These cultures were incubated at 37°C in atmosphere enriched in 5% CO₂ for 24 hours. Bacteria growth was determined as turbidity of the over night growth and was determined by computerized ELISA reader (Thermoₘₐₓ microplate reader, Molecular Devices, USA) at 650 nm. Control cultures of bacteria with no oxazaborolidines added and media broth with the oxzaborilidines added but with no bacteria were conducted. MIC was determined as the lowest concentration of the tested agent in which bacteria did not grow.

### 1.2 Results

MIC values were used to determine the antibacterial efficacy of BNO**1** to BNO**8** against *S*. *mutans* which is the one of the prediominant bacteria in the etiology of dental caries. BNO**1** and BNO**5** were most active which seems to indicate that substitution on nitrogen is desirable, while BNO**7** and BNO**8** which are formally charged showed the weakest activity.

### Minimal inhibitory concentration (MIC's) of Oxazaborolidines derivatives(#1-8) against S. mutans

| Oxazaborolidines Derivatives (#) | MIC Range (mM) | MIC Mean (mM) |
|---|---|---|
| **BNO1** | 0.77-2.33 | 1.55 |
| **BNO2** | 2-10 | 6.00 |
| **BNO3** | 2.25-4.5 | 3.38 |
| **BNO4** | 0.90-1.75 | 1.33 |
| **BNO5** | 0.26-0.80 | 0.53 |
| **BNO6** | 1.90-3.75 | 2.83 |
| **BNO7** | 4.5-9.0 | 6.75 |
| **BNO8** | 4.5-9. | 06.75 |

### Example 2: Effect of the compounds of the invention on bacterial FTF enzymatic activity

### 2.1 Experimental

Fructosyltransferase was purified as described by Rozen et al. (Rozen et al. FEMS Microbiol Lett., 2001, 195, 205-210; Rozen et al. APMIS, 2001, 109, 155-160). Briefly;a mixture of 200ul purified FTF, 100ul 1200mM sucrose supplemented with 0.3uCi ml-1 of [3H]-fructose labelled sucrose and 100ul of the tested compound at different concentrations was incubated at 37°C for 3 h. the enzymatic reaction was terminated by the addition of ice-cold ethanol to a final concentration of 70%. Ethanol-insoluble fructans were isolated by overnight precipitation at 4°C. The precipitated fructans were washed three times with ethanol and placed over 25mm glass fiber filters in multisample vacuum manifold. The filters containing the ethanol-insoluble fructans were dried by suction and placed in scintillation vials. The amount of radioactive-labeled fructans was measured in a scintillation counter.

### 2.2 Results

The results are shown in Fig. 1. (In Figure 1, BNOO1 refers to BN07, and BNOO2 refers to BNO8).

### Example 3: Effect of the compounds of the invention on bacterial adhesion to a substrate

### 3.1 Experimental

40 mg samples of hydroxyapatite (HA) beads (Ceramic hydroxyapatite type I, 80um, Bio-Rad Laboratories, Hercules, CA, USA)) (surface area, 0.63 cm²/mg) were equilibrated with 3 washes of buffered KCl to which 250 µl of labeled radioactive bacteria (prepared as described above), 400 µl of the tested boronic compound and 50 µl of 700 mM sucrose (in KCl buffer solution) were added. The mixture was incubated for 60 min at 37 C with gentle rotation. At the end of the incubation period, the beads were washed 3 times with buffered KCl for removal of unbound components, especially the non-adsorbed labeled bacteria, and then rinsed with 2 ml ethanol into vials containing 10 ml scintillation fluid (Ecoscint A, National Diagnostics, Manville, NJ). The amount of radioactively labeled bacteria adsorbed onto the HA beads was measured by scintillation counting (BETAmatic scintillation counter (Koutron®, Basel, Switzerland). Results are expressed as percent of bacterial adhesion in comparison to a control group containing no agent.

### 3.1 Results

The results are shown in Fig. 2. As can be seen, while some of the compounds decreased activity as compared to control some increased this activity.

Dose-response and structure-activity relationship (SAR), between the synthesized oxazaborilidines and anti-adhesion properties was observed at concentrations between 6-120 mM). In general: Compounds that contained B-Butyl group (BNO3, BNO5, BN06 and BN07) showed significant anti-adhesive effect ∼21-73% at their maximum tested concentration (Fig.2), while replacing the butyl by phenyl group led to an opposite effect (BNO1, BNO2, BNO4, BNO8) of increased adhesion by ∼18-62%). For example at 60mM, BN03 reduced bacterial capability to adhere to hydroxyapatite surface by 63%. Replacing B-butyl (BNO3) by a B-phenyl group (BNO2) promoted bacterial adhesion by 37% at the same above concentration
(In Figure 2, BNOO1 refers to BN07, and BNOO2 refers to BN08).

### Example 4: Effects of the compounds of the invention of quorum sensing

This experiment was designed to determine the ability of compounds of the invention to mimic or inhibit induction activity of natural A1-2.

### 4.1 Experimental

Interaction of BNO-1 and BNO-2 with AI-2 signal transduction system were examined on AI-2 reporter strain *Vibrio harveyi BB170* which was constructed to possess only AI-2 cognate sensor [Bassler, B.L. et al., Mol. Microbiol. 1993, 9, 773-786].

Bright overnight culture was diluted 1:5000 and the bioluminescence was traced along growth of the diluted bacterial culture. BNO-1 in tested concentrations was added at start of the bacterial growth. For calculation of fold induction was used the bioluminescence levels at 3 hr after start of the growth. The results are shown in Fig 3.

Bright overnight culture was diluted 1:5000 and the bioluminescence was traced along growth of the diluted bacterial culture at the presence of AI-2 which was added at start of the bacterial growth in the induced concentration. BNO-2 in tested concentrations was added at start of the bacterial growth. The residual bioluminescence is expressed as part of maximal, AI-2-inducted and non-inhibited bioluminescence. For the calculation was used bioluminescence level at 2.5 hr after start of the growth. The results are shown in Fig 4.

Luminescence was monitored by Lumac/3M Biocounter M2010, Netherlands. Culture density was measured as CFU on selective solid LM (L-marine) medium per ml of culture. Relative luminescence (*"bioluminescence"* in figures) was calculated as quotient of luminescence and culture density and expressed in relative light units (RLU, count / 10⁶ CFU).

Fold induction was calculated as relation between induced by BNO-1 and not induced relative luminescence. A measure of inhibition was defined as relation of induced by AI-2 relative luminescence in the presence and in the absence of BNO-2.

### 4.2 Results

The results show that BNO-1 induced, like AI-2, *Vibrio harveyi BB170* bioluminescence in dose dependent manner (Fig 3). In contrast, BNO-2, added together with exogenous AI-2 strongly inhibited ability of the latter to induce bioluminescence (Fig 4).

BNO-1 induced bioluminescence of *V.harveyi* BB 170 (sensor 1⁻ sensor2+).. The mechanism is via sensor 2+ as mutans with sensor 2- do not react.

BNO-2 discouraged AI-2-induced bioluminescence of *V. harveyi* BB 170 (sensor 1⁻ sensor2⁺).

### Example 5:

### Synthesis of oxazaborolidines compounds BNO1-BNO6.

The synthesis of compounds BNO1-6 is outlined in Reaction Scheme 1. One equivalent of the appropriate secondary amino alcohol and one equivalent of butyl or phenyl boronic acid were refluxed overnight in toluene and the water was excluded using Dean-Stark apparatus. The toluene was then evaporated and the residue was vacuum distilled to afford the desired compound.

The yields were BNO1: 85%, BN02: 82%, BN03: 79%, BN04: 68%, BNO5: 72%, BN06: 68%

After removal of toluene, BNO1-3 and BNO5 were isolated by distillation in high yields (85%, 82%, 79% and 72% respectively). BN04 could be obtained in high purity by recrysallization. Attempted recrystallization of BN06 gave product accompanied by some starting materials (< 5% by NMR).

In BNO1and in BNO4-6 the (-)- norpseudoephedrine derivatives were used.

### Synthesis of compounds BNO7-BNO8.

A 250-mL, one-necked, round-bottomed flask equipped with an egg-shaped magnetic stirrer was charged with one equivalent of appropriate boronic acid and one equivalent of diethanolamine. Ether and dichloromethane(1:2) were added, followed by molecular sieves 3Å. The resulting heterogeneous solution was stirred for 2 hr under argon. The solid was triturated with dichloromethane, filtered through a medium fritted disk funnel and washed with dichloromethane. The filtrate was concentrated under reduced pressure to produce the solid compound. The diethanolamine complex (solid compound) was purified by recrystallization as follows: the white solid was dissolved in hot dichloromethane, then ether was added to induce recrystallization of the solid compound. The mixture was cooled to 0°C and the solid was collected on a Buchner funnel and washed with ether. The product was dried under reduced pressure (0.2 mm) to afford the title compound as a white crystalline solid. (yield: BN07: 60%, BNO8: 45%, 16% recryst).

### Compounds Identification

### BNO1-BNO8

### NMR:

### BNO1:

¹H NMR (300 MHz, CDC1₃)_: 0.73 (d, J_{H},_{H}=6.0Hz, 3 H), 2.95 (s, 3 H), 3.81-3.84 (m, 1 H), 5.65 (d, J_{H,H}9.0Hz 1 H), 7.26-7.83 (m, 10H). ¹³C NMR (75.9 MHz, CDCl₃)_: 16.04, 31.40, 62.17, 81.92, 128-140. M.S: m/z 251.

### BNO2:

¹H NMR (300 MHz, CDCl₃) _: 1.115 (s, 6 H), 3.37 (br s, 1 H), 3.60 (s, 1 H), 7.26-7.97 (m, 5H). ¹³C NMR (75.9 MHz, CDCl₃) _: 26.24, 54.58, 72.76, 127.53, 128.79, 130.17, 132.20, 133.77. M.S: m/z 175.

### BNO3:

¹H NMR (300 MHz,D₂O) _: 0.44 (t 2H), 0.71 (t, J_{H,H}=9.0Hz, 3H), 0.94 (s, 6H), 1.04-1.13 (m, 4H), 3.21(s, 2H). ¹³C NMR (75.9 MHz, D₂O) _: 13.41, 24.15, 25.28, 26.51, 51.36, 70.07. M.S: m/z 155.

### BNO4:

¹H NMR (300 MHz, CDCl₃) _: 1.38 (d, J_{H,H}=6.0Hz, 3 H), 3.71-3.75 (m, 1 H), 3.88 (br s, 1 H), 5.02 (d, J_{H,H}=6.0Hz, 1 H), 7.29-7.78 (m, 10 H). M.S: m/z 237.

### BNO5:

¹H NMR (300 MHz, CDCl₃) _: 0.60 (d, 3 H), 0.82-0.90 (t, overlap, 5 H), 1.34-1.50 (m, 4 H), 2.66 (s, 3 H), 3.60-3.75 (m, 1 H), 5.45 (d, 1 H), 7.23-7.40 (m, 5 H). M.S: m/z 231.

### BNO6:

¹³C NMR (75.9 MHz, CDCl₃) _: 16.19, 21.60, 25.85, 26.61, 28.30, 57.05, 82.11, 127.06,128.27, 128.67. M.S: m/z 217.

### BNO7:

¹H NMR (300 MHz, CDCl₃) _: 0.43-0.46 (m, 2 H), 0.88 (t, 3 H), 1.250-1.33 (m, 4 H), 2.75 (br s, 2 H), 3.27 (br s, 2 H), 3.83-3. 96 (m, 4H), 5.83 (br s, 1 H). ¹³C NMR (75.9 MHz, CDCl₃) _: 14.45, 18.43, 26.80, 28.49, 51.84, 62.91.

### BNO8:

¹H NMR (300 MHz, DMSO) _: 2.79- 2.84 (m, 2 H), 3.03-3.09 (m, 2 H), 3.73-3.87 (overlap, 4 H), 6.8 (br s, 1 H), 7.12-7.18 (overlap, 3 H), 7.42 (d, J_{H,H}=9.0Hz, 2 H).

### Example 6: BNO1-BNO5 Hydrates

The following hydrate compounds were prepared:
B-hydroxy-3,4-dimethyl-2,5-diphenyl-1,3,2-oxazaborolidine (BNO1 I hydrate);
B-hydroxy-4,4-dimethyl-2-phenyl-1,3,2-oxazaborolidine (BNO2 hydrate);
B-hydroxy-2-butyl-4,4-dimethyl-1,3,2-oxazaborolidine (BNO3 hydrate);
B-hydroxy-4-methyl-2,5-diphenyl-1,3,2-oxazaborolidine (BNO4 hydrate);
B-hydroxy-2-butyl-3,4-dimethyl-5-phenyl-1,3,2-oxazaborolidine (BNO5 hydrate);

The hydrate compounds were obtained by dissolving the appropriate oxazaborolidine in water and stirring for 2 hours.

### The effect of pH on the oxazaborolidine derivatives

The oxazaborolidines derivatives with B-n-butyl group are more stable at neutral and acidic pH's, but at pH∼10.6, -90% of an intermediate is formed which is a result of an equilibrium reaction of the oxazaborolidines with H₂O (Reaction scheme II)

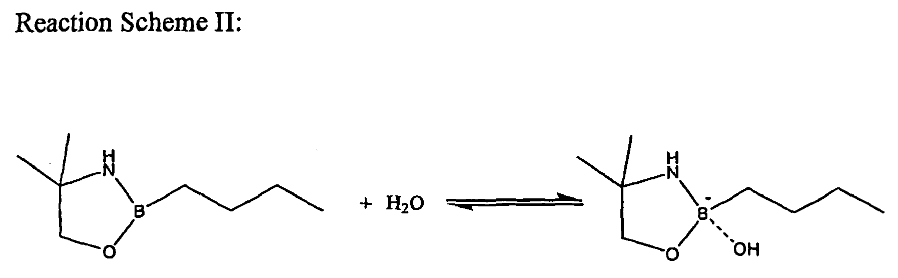

At more basic conditions, there is complete conversion of the oxazaborolidine to a complex (ate-complex) and this is corroborated by ¹¹B NMR that shows a peak with chemical shift of ∼ 5.2ppm.

At pH=10.8, the free n-butylboronic acid has a chemical shift of 19.974ppm, while BN03, the B-butyl containing derivative gives two peaks, one at 24.868ppm (90%) which is the intermediate and a peak at 6.8ppm (10%) that belongs to the mentioned above complex. At further basic conditions, a peak at 5.2ppm exist which indicate a complete conversion to an ate-complex moiety.

This compound (BN03) in chloroform solvent and at neutral pH, gives one peak at 33.667ppm that belongs to the oxazaborolidines-. On the other hand the B-phenyl containing derivative (BN02) in the same solvent gives two peaks: one at 31.268ppm and other at 7.404 indicative of the product and a complex respectively.

On the other hand, the B-phenyl containing derivatives are converted to the ate- complex once they are fully dissolved in H₂O, the ate-complex appears at 5.461ppm in the ¹¹B NMR.

Thus we assume that the conversion to the ate complex is faster for the phenyl containing derivatives compared to the butyl derivatives

### Compounds Identification

### NMR:

### BNO1 (hydrate):

¹¹B NMR (96.2 MHz) δ: 7.18.

### BNO2 (hydrate):

¹H NMR (300 MHz, D₂O) δ: 1.08 (s, 6 H), 3.34 (br s, 1 H), 4.65 (s, 1 H. overlapped with D20), 7.15-7.46 (m, 5H)
¹³C NMR (75.9 MHz, D₂O) 8: 14.20, 22.29, 54.39; 66.11, 67.39, 127.63, 132.04
¹¹B NMR (96.2 MHz) δ: 6.032

### BNO3(hydrate):

¹H NMR (300 MHz,D₂O) δ: 0.44 (t, 2H), 0.71 (t, *J*_{H,H}=9.0H_{Z,} 3H), 0.94 (s, 6H), 1.04-1.13 (m, 4H). 3.21(s, 2H). ¹³C NMR (75.9 MHz, D₂O) δ: 13.41, 24.15, 25.28, 26.51, 51.36, 70.07. ¹¹B NMR (96.2 MHz) 8: 5.2

### BNO4 (hydrate):

¹H NMR (300 MHz, D₂O) δ: 0.86 (d, *J*_{H,H}=6.6H_{Z}, 3 H), 3.06-3.27 (m. 1 H), 3.05 (br s, 1 H), 4.36(d, *J*_{H,H}=8.0H_{Z,} 1 H), 7.16-7.51 (m, 10 H).
¹¹B NMR (96.2 MHz) δ: 4.47

### BNO5(hydrate):

¹¹B NMR (96.2 MHz) δ:, 5.98

## Claims

1. A composition for modulation of at least one bacteria-related parameter selected from:
(a) adhesion of the bacteria to its substrate;
(b) enzymatic activity of the enzymes;
(c) viability of the bacteria;
(d) effect on quorum sensing;
(e) biofilm formation by the bacteria;
(f) a combination of two or more of (a)-(e);
comprising a compound of formula I including a pharmaceutically acceptable salt, solvate, hydrate, isomers, and stereoisomers thereof: 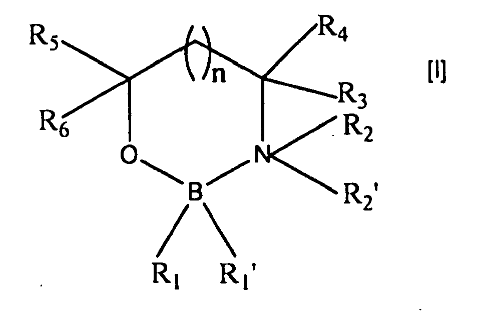 wherein,
n = 0, 1, 2, 3;
R₁ is selected from hydrogen, C₁-C₈alkyl, C₂-C₈ alkenyl, aryl, and C₃-C₇cycloalkyl;
R₂ is selected from hydrogen, C₁-C₈alkyl, C₂-C₈ alkenyl, aryl, C₃-C₇cycloalkyl, -(C=O)R, and -S(=O)₂R, where R is selected from C₁-C₈alkyl, aryl, and C₃-C₇cycloalkyl;
R₃ and R₄ are each independently selected from hydrogen, C₁-C₈alkyl, C₂-C₈alkenyl, aryl, and C₃-C₇cycloalkyl;
R₅ and R₆ are each independently selected from hydrogen, C₁-C₈alkyl, C₂-C₈ alkenyl, aryl, and C₃-C₇cycloalkyl;
or one of R₃ and R₄ together with one of R₅ and R₆ form a substituted or unsubstituted aromatic ring, a substituted or unsubstituted cycloalkyl ring, or a substituted or unsubstituted heterocyclic ring, fused to the oxazaborolidine ring,
wherein the substituent of said substituted aromatic, substituted cycloalkyl, or substituted heterocyclic ring is at least one substituent selected from hydroxy, halo, and C₁-C₆ alkyl groups,
R₁' is selected from null, hydrogen, hydroxyl, C₁-C₈alkyl, C₂-C₈ alkenyl and aryl;
R₂' is selected from null, hydrogen, C₁-C₈alkyl, C₂-C₈ alkenyl and aryl;
or R₁' together with R₂' are a group selected from -OR₁₀ -, -O-(C=O)R₁₀-, and -O-R₁₀(C=O)-, wherein R₁₀ is selected from a substituted or unsubstituted C₁-C₃alkyl, a substituted or unsubstituted aryl and a substituted or unsubstituted heteroaryl, thereby forming a ring fused to the oxazaborolidine ring,
wherein the substituent of said substituted C₁-C₃alkyl, substituted aryl or substituted heteroaryl is at least one substituent selected from hydroxy, halo and C₁-C₆ alkyl groups.

2. The composition of claim 1 wherein n = 0.

3. The composition of claim 1 wherein one of R₃ and R₄ together with one of R₅ and R₆ are -(CH₂)X(CH₂)ₚ- , wherein p = 1, 2, 3, X = O, S, N-R', N(C=O)R", wherein R' is selected from hydrogen, C₁-C₈alkyl, C₂-C₈alkenyl, C₃-C₇cycloalkyl, and aryl, R" is selected from C₁-C₈alkyl, C₂-C₈alkenyl, C₃-C₇cycloalkyl, and aryl, thereby forming a heterocyclic ring fused to the oxazaborolidine ring.

4. The composition of claim 1 wherein n = 0, R₁ is selected from C₁-C₈alkyl and aryl; R₂ is selected from hydrogen and C₁-C₈alkyl; R₃ and R₄ are each independently selected from hydrogen, C₁-C₈alkyl and aryl; R₅ and R₆ are each independently selected from hydrogen, C₁-C₈ alkyl and aryl; R₁' is selected from null and hydroxyl; R₂' is selected from null and hydrogen; or R₁' together with R₂' are a -OR₁₀- group, wherein R₁₀ is C₁-C₃ alkyl, thereby forming a heterocyclic ring fused to the oxazaborolidine ring.

5. The composition of claim 1 or 4 wherein n = 0, R₁ is selected from C₁-C₈alkyl and aryl; R₂ is selected from hydrogen and C₁-C₈alkyl; R₃ and R₄ are each independently selected from hydrogen, C₁-C₈alkyl and aryl; R₅ and R₆ are each independently selected from hydrogen, C₁-C₈ alkyl and aryl; R₁' is selected from null and hydroxyl; and R₂' is selected from null and hydrogen.

6. The composition of claim 5, wherein said compound of formula I is selected from:
3,4-dimethyl-2,5-diphenyl-1,3,2-oxazaborolidine;
4,4-dimethyl-2-phenyl-1,3,2-oxazaborolidine;
2-butyl-4,4-dimethyl-1,3,2-oxazaborolidine;
4-methyl-2,5-diphenyl-1,3,2-oxazaborolidine;
2-butyl-3,4-dimethyl-5-phenyl-1,3,2-oxazaborolidine;
2-butyl-4-methyl-5-phenyl-1,3,2-oxazaborolidine;
B-hydroxy-3,4-dimethyl-2,5-diphenyl-1,3,2-oxazaborolidine;
B-hydroxy-4,4-dimethyl-2-phenyl-1,3,2-oxazaborolidine;
B-hydroxy-2-butyl-4,4-dimethyl-1,3,2-oxazaborolidine;
B-hydroxy-4-methyl-2,5-diphenyl-1,3,2-oxazaborolidine;
B-hydroxy-2-butyl-3,4-dimethyl-5-phenyl-1,3,2-oxazaborolidine; and B-hydroxy-2-butyl-4-methyl-5-phenyl-1,3,2-oxazaborolidine.

7. The composition of claim 1 or 4 wherein n = 0, R₁ is selected from C₁-C₈ alkyl and aryl; R₂ is selected from hydrogen and C₁-C₈alkyl; R₃ and R₄ are each independently selected from hydrogen, C₁-C₈alkyl and aryl; R₅ and R₆ are each independently selected from hydrogen, C₁-C₈alkyl and aryl; and R₁' together with R₂' are a -OR₁₀- group, wherein R₁₀ is C₁-C₃ alkyl, thereby forming a heterocyclic ring fused to the oxazaborolidine ring.

8. The composition of claim 7 wherein said compound is selected from:
[(2-)-N,O,O'[2,2'-Iminobis[ethanolato]]]-2-n-butylboron; and
[(2-)-N,O,O'[2,2'-Iminobis[ethanolato]]]-2-phenylboron.

9. The composition of claim 1 wherein said modulation is decrease of said bacteria-related parameter.

10. The composition of claim 1 wherein said bacteria-related parameter is adhesion of the bacteria to its substrate and said modulation is increase.

11. The composition of claim 1 wherein said bacteria related parameter is viability of bacteria, said modulation is decrease and said compound is selected from 3,4-dimethyl-2,5-diphenyl-1,3,2-oxazaborolidine; and 2-butyl-3,4-dimethyl-5-phenyl-1,3,2-oxazaborolidine.

12. The composition of claim 1 wherein said bacteria related parameter is adhesion of the bacteria to its substrate, said modulation is decrease, and said compound is selected from 2-butyl-4,4-dimethyl-1,3,2-oxazaborolidine, 2-butyl-3,4-dimethyl-5-phenyl-1,3,2-oxazaborolidine, 2-butyl-4-methyl-5-phenyl-1,3,2-oxazaborolidine, and [(2-)-N,O,O'[2,2'-iminobis[ethanolato]]]-2-n-butylboron.

13. The composition of claim 1 wherein said bacteria related parameter is adhesion of the bacteria to its substrate, said modulation is increase, and said compound is selected from 3,4-dimethyl-2,5-diphenyl-1,3,2-oxazaborolidine, 4,4-dimethyl-2-phenyl-1,3,2-oxazaborolidine, 4-methyl-2,5-diphenyl-1,3,2-oxazaborolidine, and [(2-)-N,O,O'[2,2'-Iminobis[ethanolato]]]-2-phenylboron.

14. The composition of claim 1 wherein said bacteria related parameter is effect on quorum sensing, said modulation is increase, and said compound is 3,4-dimethyl-2,5-diphenyl-1,3,2-oxazaborolidine.

15. The composition of claim 1 wherein said bacteria related parameter is effect on quorum sensing, said modulation is decrease, and said compound is 4,4-dimethyl-2-phenyl-1,3,2-oxazaborolidine.

16. A compound of formula I including a pharmaceutically acceptable salt, solvate, hydrate, isomers, and stereoisomers thereof: 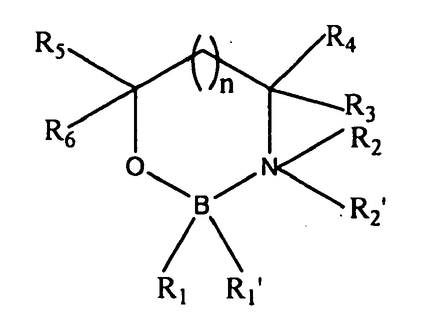 wherein,
n=0;
R₁ is selected from C₁-C₈alkyl and aryl;
R₂ is selected from hydrogen and C₁-C₈alkyl;
R₃ and R₄ are each independently selected from hydrogen, C₁-C₈alkyl and aryl;
R₅ and R₆ each independently selected from hydrogen, C₁-C₈alkyl and aryl; and
R₁' together with R₂' are -OR₁₀- group, wherein R₁₀ is a C₁-C₃alkyl, thereby forming a heterocyclic ring fused to the oxazaborolidine ring;
with the proviso that the following compounds are excluded wherein
R₁ is C₂-C₄alkyl group or C₆alkyl group, R₁' together with R₂' are -OR₁₀- group, wherein R₁₀ is C₂alkyl group, and R₂ to R₆ represent a hydrogen atom; and
R₁ is C₆alkyl group, R₁' together with R₂' are -OR₁₀- group, wherein R₁₀ is C₂alkyl group, R₂ is a C₁alkyl group and R₃ to R₆ represent a hydrogen atom.

17. The compound of claim 16 wherein R₁₀ is ethyl.

18. The compound of claim 16 wherein said R₁ is aryl.

19. The compound of claim 16 wherein said aryl of R₁ is a phenyl.

20. The compound of claim 16 wherein said C₁-C₈alkyl is C₁-C₄alkyl.

21. The compound of claim 16 wherein said R₁ is C₁-C₈alkyl.

22. The compound of claim 16 wherein said R₁ is C₁-C₄alkyl.

23. The compound of claim 16 wherein said C₁-C₈alkyl of R₁ is a butyl.

24. The compound of claim 16 wherein said R₂ is hydrogen.

25. The compound of claim 16 wherein said R₂ is C₁-C₈alkyl.

26. The compound of claim 16 wherein said C₁-C₈alkyl of R₂ is methyl.

27. The compound of claim 16 wherein R_{3,} R₄, R₅ and R₆ are hydrogen.

28. The compound of claim 16 being [(2-)-N,O,O'[2,2'-iminobis[ethanolato]]]-2-phenylboron.

29. A composition for decreasing bacterial growth comprising a compound of formula (I) as defined in any one of claims 1-28.

30. A composition for increasing the susceptibility of bacteria to the cytotoxic effects of other antibacterial agents comprising the compound of formula (I) as defined in any one of claims 1-28.

31. A pharmaceutical composition comprising a pharmaceutically acceptable carrier, and as an active ingredient a compound having formula I as defined in any one of claims 1-28.

32. The pharmaceutical composition of claim 31, wherein said composition is for the treatment, prevention, or amelioration of bacterial infection.

33. The pharmaceutical composition of claim 31, wherein said composition is for increasing the susceptibility of bacteria to the cytotoxic effect of other antibacterial compounds.

34. The pharmaceutical composition of claim 31 further comprising at least one other antibacterial agent.

35. Use of the compounds of formula I as defined in any one of claims 1-28 for the preparation of a pharmaceutical preparation for decreasing bacterial growth.

## Patentansprüche

1. Zusammensetzung zur Modulation von wenigstens einem bakterienbezogenen Parameter ausgewählt aus:
(a) Adhäsion der Bakterien auf ihr Substrat;
(b) enzymatische Aktivität der Enzyme;
(c) Lebensfähigkeit der Bakterien;
(d) Wirkung auf Quorum Sensing;
(e) Biofilmbildung durch die Bakterien;
(f) eine Kombination aus zweien oder mehreren von (a)-(e);
umfassend eine Verbindung der Formel I beinhaltend ein pharmazeutisch akzeptables Salz, Solvat, Hydrat, Isomere und Stereoisomere davon: 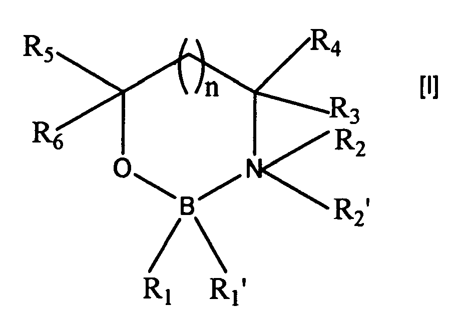 worin
n = 0, 1, 2, 3;
R₁ ausgewählt ist aus Wasserstoff, C₁-C₈-Alkyl, C₂-C₈-Alkenyl, Aryl und C₃-C₇-Cycloalkyl;
R₂ ausgewählt ist aus Wasserstoff, C₁-C₈-Alkyl, C₂-C₈-Alkenyl, Aryl, C₃-C₇-Cycloalkyl, -(C=O)R und -S(=O)₂R, worin R ausgewählt ist aus C₁-C₈-Alkyl, Aryl und C₃-C₇-Cycloalkyl;
R₃ und R₄ jeweils unabhängig voneinander ausgewählt sind aus Wasserstoff, C₁-C₈-Alkyl, C₂-C₈-Alkenyl, Aryl und C₃-C₇-Cycloalkyl;
R₅ und R₆ jeweils unabhängig voneinander ausgewählt sind aus Wasserstoff, C₁-C₈-Alkyl, C₂-C₈-Alkenyl, Aryl und C3-C₇-Cycloalkyl;
oder eines von R₃ und R₄ zusammen mit einem von R₅ und R₆ einen substituierten oder nichtsubstituierten aromatischen Ring, einen substituierten oder nichtsubstituierten Cycloalkylring, oder einen substituierten oder nichtsubstituierten heterocyclischen Ring bildet, der an den Oxazaborolidinring annelliert ist,
worin der Substituent des substituierten aromatischen, substituierten Cycloalkyl- oder substituierten heterocyclischen Rings wenigstens ein Substituent ausgewählt aus Hydroxy, Halogen und C₁-C₆-Alkylgruppen ist,
R₁' ausgewählt ist aus nicht vorhanden, Wasserstoff, Hydroxyl, C₁-C₈-Alkyl, C₂-C₈-Alkenyl und Aryl;
R₂' ausgewählt ist aus nicht vorhanden, Wasserstoff, C₁-C₈-Alkyl, C₂-C₈-Alkenyl und Aryl; oder R₁' zusammen mit R₂' eine Gruppe sind ausgewählt aus -OR₁₀-, -O-(C=O)R₁₀-, und -O-R₁₀(C=O)-, worin R₁₀ ausgewählt ist aus einem substituierten oder nichtsubstituierten C₁-C₃-Alkyl, einem substituierten oder nichtsubstituierten Aryl und einem substituierten oder nichtsubstituierten Heteroaryl, wodurch sie einen an den Oxazaborolidinring annellierten Ring bilden,
worin der Substituent des substituierten C₁-C₃Alkyl, substituierten Aryl oder substituierten Heteroaryl wenigstens ein Substituent ausgewählt aus Hydroxy, Halogen und C₁-C₆-Alkylgruppen ist.

2. Die Zusammensetzung gemäß Anspruch 1, worin n = 0.

3. Die Zusammensetzung gemäß Anspruch 1, worin eines von R₃ und R₄ zusammen mit einem von R₅ und R₆ -(CH₂)X(CH₂)ₚ- sind, worin p = 1, 2, 3, X = O, S, N-R', N(C=O)R", worin R' ausgewählt ist aus Wasserstoff, C₁-C₈-Alkyl, C₂-C₈-Alkenyl, C₃-C₇-Cycloalkyl, und Aryl, R" ausgewählt ist aus C₁-C₈-Alkyl, C₂-C₈-Alkenyl, C₃-C₇-Cycloalkyl und Aryl, wodurch sie einen heterocyclischen Ring bilden, der an den Oxazaborolidinring annelliert ist.

4. Die Zusammensetzung gemäß Anspruch 1, worin n = 0, R₁ ausgewählt ist aus C₁-C₈-Alkyl und Aryl; R₂ ausgewählt ist aus Wasserstoff und C₁-C₈-Alkyl; R₃ und R₄ jeweils unabhängig ausgewählt sind aus Wasserstoff, C₁-C₈-Alkyl und Aryl; R₅ und R₆ jeweils unabhängig ausgewählt sind aus Wasserstoff, C₁-C₈-Alkyl und Aryl; R₁' ausgewählt ist aus nicht vorhanden und Hydroxyl; R₂' ausgewählt ist aus nicht vorhanden und Wasserstoff; oder R₁' zusammen mit R₂' eine -OR₁₀- Gruppe sind, worin R₁₀ C₁-C₃-Alkyl ist, wodurch ein heterocyclischer Ring gebildet wird, der an den Oxazaborolidinring annelliert ist.

5. Die Zusammensetzung gemäß Anspruch 1 oder 4, worin n = 0, R₁ ausgewählt ist aus C₁-C₈-Alkyl und Aryl; R₂ ausgewählt ist aus Wasserstoff und C₁-C₈-Alkyl; R₃ und R₄ jeweils unabhängig ausgewählt sind aus Wasserstoff, C₁-C₈-Alkyl und Aryl; R₅ und R₆ jeweils unabhängig ausgewählt sind aus Wasserstoff, C₁-C₈-Alkyl und Aryl; R₁' ausgewählt ist aus nicht vorhanden und Hydroxyl; und R₂' ausgewählt ist aus nicht vorhanden und Wasserstoff.

6. Die Zusammensetzung gemäß Anspruch 5, worin die Verbindung der Formel ausgewählt ist aus:
3,4-Dimethyl-2,5-diphenyl-1,3,2-oxazaborolidin;
4,4-Dimethyl-2-phenyl-1,3,2-oxazaborolidin;
2-Butyl-4,4-dimethyl-1,3,2-oxazaborolidin;
4-Methyl-2,5-diphenyl-1,3,2-oxazaborolidin;
2-Butyl-3,4-dimethyl-5-phenyl-1,3,2-oxazaborolidin;
2-Butyl-4-methyl-5-phenyl-1,3,2-oxazaborolidin;
B-Hydroxy-3,4-dimethyl-2,5-diphenyl-1,3,2-oxazaborolidin;
B-Hydroxy-4,4-dimethyl-2-phenyl-1,3,2-oxazaborolidin;
B-Hydroxy-2-butyl-4,4-dimethyl-1,3,2-oxazaborolidin;
B-Hydroxy-4-methyl-2,5-diphenyl-1,3,2-oxazaborolidin;
B-Hydroxy-2-butyl-3,4-dimethyl-5-phenyl-1,3,2-oxazaborolidin; und B-Hydroxy-2-butyl-4-methyl-5-phenyl-1,3,2-oxazaborolidin.

7. Die Zusammensetzung gemäß Anspruch 1 oder 4, worin n = 0, R₁ ausgewählt ist aus C₁-C₈-Alkyl und Aryl; R₂ ausgewählt ist aus Wasserstoff und C₁-C₈-Alkyl; R₃ und R₄ jeweils unabhängig ausgewählt sind aus Wasserstoff, C₁-C₈-Alkyl und Aryl; R₅ und R₆ jeweils unabhängig ausgewählt sind aus Wasserstoff, C₁-C₈-Alkyl und Aryl; und R₁' zusammen mit R₂' eine -OR₁₀- Gruppe sind, worin R₁₀ C₁-C₃-Alkyl ist, wodurch ein heterocyclischer Ring gebildet wird, der an den Oxazaborolidinring annelliert ist.

8. Die Zusammensetzung gemäß Anspruch 7, worin die Verbindung ausgewählt ist aus:
[(2-)-N,O,O'[2,2'-Iminobis[ethanolato]]]-2-n-butylboron; und
[(2-)-N,O,O'[2,2'-Iminobis[ethanolato]]]-2-phenylboron.

9. Die Zusammensetzung gemäß Anspruch 1, worin die Modulation eine Abnahme des bakterienbezogenen Parameter ist.

10. Die Zusammensetzung gemäß Anspruch 1, worin der bakterienbezogene Parameter eine Adähsion der Bakterien auf ihr Substrat ist und die Modulation eine Zunahme ist.

11. Die Zusammensetzung gemäß Anspruch 1, worin der bakterienbezogene Parameter die Lebensfähigkeit der Bakterien ist, die Modulation eine Abnahme ist und die Verbindung ausgewählt ist aus 3,4-Dimethyl-2,5-diphenyl-1,3,2-oxazaborolidin; und 2-Butyl-3,4-dimethyl-5-phenyl-1,3,2-oxazaborolidin.

12. Die Zusammensetzung gemäß Anspruch 1, worin der bakterienbezogene Parameter die Adhäsion der Bakterien auf ihr Substrat ist, die Modulation eine Abnahme ist und die Verbindung ausgewählt ist aus 2-Butyl-4,4-dimethyl-1,3,2-oxazaborolidin, 2-Butyl-3,4-dimethyl-5-phenyl-1,3,2-oxazaborolidin, 2-Butyl-4-methyl-5-phenyl-1,3,2-oxazaborolidin, und [(2-)-N,O,O'[2,2'-lminobis[ethanolato]]]-2-n-butylboron.

13. Die Zusammensetzung gemäß Anspruch 1, worin der bakterienbezogene Parameter die Adhäsion der Bakterien auf ihr Substrat ist, die Modulation eine Zunahme ist und die Verbindung ausgewählt ist aus 3,4-Dimethyl-2,5-diphenyl-1,3,2-oxazaborolidin, 4,4-Dimethyl-2-phenyl-1,3,2-oxazaborolidin, 4-Methyl-2,5-diphenyl-1,3,2-oxazaborolidin und [(2-)-N,O,O'[2,2'-Iminobis[ethanolato]]]-2-phenylboron.

14. Die Zusammensetzung gemäß Anspruch 1, worin der bakterienbezogene Paramter die Wirkung auf das Quorum Sensing ist, die Modulation eine Zunahme ist und die Verbindung 3,4-Dimethyl-2,5-diphenyl-1,3,2-oxazaborolidin ist.

15. Die Zusammensetzung gemäß Anspruch 1, worin der bakterienbezogene Parameter die Wirkung auf das Quorum Sensing ist, die Modulation eine Abnahme ist und die Verbindung 4,4-Dimethyl-2-phenyl-1,3,2-oxazaborolidin ist.

16. Verbindung der Formel I beinhaltend ein pharmazeutisch akzeptables Salz, Solvat, Hydrat, Isomere und Stereoisomere davon: 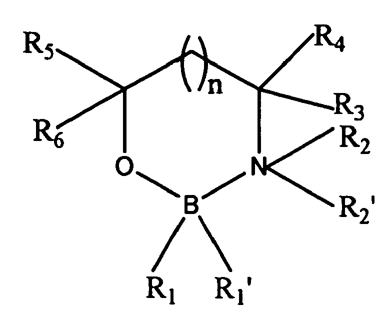 worin
n = 0;
R₁ ausgewählt ist aus C₁-C₈-Alkyl und Aryl;
R₂ ausgewählt ist aus Wasserstoff und C₁-C₅-Alkyl;
R₃ und R₄ jeweils unabhängig ausgewählt sind aus Wasserstoff. C₁-C₈-Alkyl und Aryl;
R₅ and R₆ jeweils unabhängig ausgewählt sind aus Wasserstoff, C₁-C₈-Alkyl und Aryl; und
R₁' zusammen mit R₂' eine -OR₁₀- Gruppe sind, worin R₁₀ ein C₁-C₃-Alkyl ist, wodurch ein heterocyclischer Ring gebildet wird, der an den Oxazaborolidinring annelliert ist;
mit der Maßgabe, dass die folgenden Verbindungen ausgeschlossen sind, worin
R₁ C₂-C₄-Alkylgruppe oder C₆-Alkylgruppe ist, R₁' zusammen mit R₂' eine -OR₁₀- Gruppe ist,
worin R₁₀ C₂-Alkylgruppe ist, und R₂ bis R₆ ein Wasserstoffatom bedeuten; und
R₁ C₆-Alkylgruppe ist, R₁' zusammen mit R₂' eine -OR₁₀- Gruppe sind, worin R₁₀ C₂-Alkylgruppe sind, R₂ eine C₁-Alkylgruppe ist und R₃ bis R₆ ein Wasserstoffatom bedeuten.

17. Die Verbindung gemäß Anspruch 16, worin R₁₀ Ethyl ist.

18. Die Verbindung gemäß Anspruch 16, worin R₁ Aryl ist.

19. Die Verbindung gemäß Anspruch 16, worin das Aryl von R₁ ein Phenyl ist.

20. Die Verbindung gemäß Anspruch 16, worin das C₁-C₈-Alkyl ein C₁-C₄-Alkyl ist.

21. Die Verbindung gemäß Anspruch 16, worin R₁ C₁-C₈-Alkyl ist.

22. Die Verbindung gemäß Anspruch 16, worin R₁ C₁-C₄-Alkyl ist.

23. Die Verbindung gemäß Anspruch 16, worin das C₁-C₈-Alkyl von R₁ ein Butyl ist.

24. Die Verbindung gemäß Anspruch 16, worin R₂ Wasserstoff ist.

25. Die Verbindung gemäß Anspruch 16, worin R₂ C₁-C₈-Alkyl ist.

26. Die Verbindung gemäß Anspruch 16, worin das C₁-C₈-Alkyl von R₂ ein Methyl ist.

27. Die Verbindung gemäß Anspruch 16, worin R₃, R₄, R₅ und R₆ Wasserstoff sind.

28. Die Verbindung gemäß Anspruch 16, welche [(2-)-N,O,O'[2,2'-lminobis[ethanolato]]]-2-phenylboron ist.

29. Zusammensetzung zur Abnahme von Bakterienwachstum, umfassend eine Verbindung der Formel (I) wie in einem der Ansprüche 1-28 definiert.

30. Zusammensetzung zur Zunahme der Empfindlichkeit von Bakterien auf die zytotoxischen Wirkungen von anderen antibakteriellen Mitteln, umfassend die Verbindung gemäß Formel (I) wie in einem der Ansprüche 1-28 definiert.

31. Pharmazeutische Zusammensetzung, umfassend einen pharmazeutisch akzeptablen Träger und als Wirkstoff eine Verbindung mit der Formel (I) wie in einem der Ansprüche 1-28 definiert.

32. Die pharmazeutische Zusammensetzung gemäß Anspruch 31, worin die Zusammensetzung zur Behandlung, Vorbeugung oder Verbesserung von bakteriellen Infektionen ist.

33. Die pharmazeutische Zusammensetzung gemäß Anspruch 31, worin die Zusammensetzung zur Erhöhung der Empfindlichkeit der Bakterien auf die zytotoxische Wirkung von anderen antibakteriellen Verbindungen ist.

34. Die pharmazeutische Zusammensetzung gemäß Anspruch 31, weiterhin umfassend wenigstens ein anderes antibakterielles Mittel.

35. Verwendung der Verbindungen gemäß Formel 1, wie in einem der Ansprüche 1-28 definiert, zur Herstellung einer pharmazeutischen Zubereitung zur Abnahme von Bakterienwachstum.

## Revendications

1. Une composition pour la modulation d'au moins un paramètre relié à une bactérie sélectionné parmi:
(a) l'adhésion d'une bactérie à son substrat;
(b) l'activité enzymatique des enzymes;
(c) la viabilité de la bactérie;
(d) l'effet sur le quorum sensing;
(e) la formation d'un biofilm par la bactérie;
(f) une combinaison de deux ou plus de (a)-(e);
comprenant un composé de formule I incluant un sel pharmaceutiquement acceptable, le solvate, l'hydrate, les isomères, et stéréo-isomères de celui-ci: 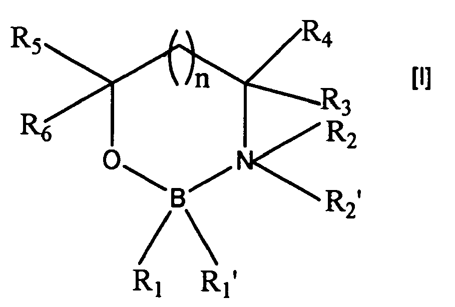 dans lequel,
n = 0, 1, 2, 3;
R₁ est sélectionné parmi l'hydrogène, un alkyle en C₁-C₈, un alcène en C₂-C₈ , un aryle, et un cycloalkyle en C₃-C₇;
R₂ est sélectionné parmi l'hydrogène, un alkyle en C₁-C₈, un alcène en C₂-C₈, un aryle, un cycloalkyle en C₃-C₇, -(C=O)R, et -S(=O)₂R, où R est sélectionné parmi un alkyle en C₁-C₈, un aryle, un cycloalkyle en C₃-C_{7 ;}
R₃ et R₄ sont chacun sélectionnés indépendamment parmi l'hydrogène, un alkyle en C₁-C₈, un alcène en C₂-C₈ , un aryle, et un cycloalkyle en C₃-C₇;
R₅ and R₆ sont chacun sélectionnés indépendamment parmi l'hydrogène, un alkyle en C₁-C₈, un alcène en C₂-C₈, un aryle, et un cycloalkyle en C₃-C₇;
ou un parmi R₃ et R₄ ensemble avec un parmi R₅ et R₆ forment un cycle aromatique substitué ou non-substitué, un cycloalkyle substitué ou non-substitué, ou un cycle hétérocyclique substitué ou non-substitué, fusionné avec le cycle oxazaborolidine,
dans lequel le substituent dudit aromatique, cycloalkyle, ou heterocycle substitué est au moins un substituant sélectionné parmi les groupes hydroxyle, halogène et alkyle en C₁-C₆,
R₁' est sélectionné parmi nul, l'hydrogène, l'hydroxyle, un alkyle en C₁-C₈, un alcène en C₂-C₈ et un aryle;
R₂' est sélectionné parmi nul, l'hydrogène, un alkyle en C₁-C₈, un alcène en C₂-C₈ et un aryle;
ou R₁' ensemble avec R₂' sont un groupe sélectionné parmi -OR₁₀ -, -O-(C=O)R₁₀-, et -O-R₁₀(C=O)-, dans lequel R₁₀ est sélectionné parmi un alkyle en C₁-C₃ substitué ou non-substitué, un aryle substitué ou non-substitué et un hétéroaryle substitué ou non-substitué, formant ainsi un cycle fusionné au cycle oxazaborolidine,
dans lequel le substituant dudit alkyle en C₁-C₃ , aryle ou hétéroaryle substitué est au moins un substituant sélectionné parmi les groupes hydroxyle, halogène et alkyle en C₁-C₆.

2. La composition selon la revendication 1 dans laquelle n = 0.

3. La composition selon la revendication 1 dans laquelle un parmi R₃ et R₄ ensemble avec un parmi R₅ et R₆ sont -(CH₂)X(CH₂)ₚ- , dans lequel p = 1, 2, 3, X = O, S, N-R', N(C=O)R", dans lequel R' est sélectionné parmi l'hydrogène, un alkyle en C₁-C₈, un alcène en C₂-C₈, un cycloalkyle en C₃-C₇, et un aryle, R" est sélectionné parmi un alkyle en C₁-C₈, un alcène en C₂-C₈, un cycloalkyle en C₃-C₇, et un aryle, formant ainsi un hétérocycle fusionné avec le cycle oxazaborolidine.

4. La composition selon la revendication 1 dans laquelle n = 0, R₁ est sélectionné parmi un alkyle en C₁-C₈ et un aryle; R₂ est sélectionné parmi l'hydrogène et un alkyle en C₁-C₈; R₃ et R₄ sont chacun indépendamment sélectionnés parmi l'hydrogène, un alkyle en C₁-C₈ et un aryle; R₅ et R₆ sont chacun indépendamment sélectionnés parmi l'hydrogène, un alkyle en C₁-C₈ et un aryle; R₁' est sélectionné parmi nul et l'hydroxyle; R₂' est sélectionné parmi nul et l'hydrogène; ou R₁' ensemble avec R₂' sont un groupe -OR₁₀-, dans lequel R₁₀ est un alkyle en C₁-C₃, formant ainsi un hétérocycle fusionné au cycle oxazaborolidine.

5. La composition selon la revendication 1 ou 4 dans laquelle n = 0, R₁ est sélectionné parmi un alkyle en C₁-C₈ et un aryle; R₂ est sélectionné parmi l'hydrogène et un alkyle en C₁-C₈; R₃ et R₄ sont chacun indépendamment sélectionnés parmi l'hydrogène, un alkyle en C₁-C₈ et un aryle; R₅ et R₆ sont chacun indépendamment sélectionnés parmi l'hydrogène, un alkyle en C₁-C₈ et un aryle; R₁' est sélectionné parmi nul et l'hydroxyle; R₂' est sélectionné parmi nul et l'hydrogène.

6. La composition selon la revendication 5 dans laquelle ledit composé de Formule I est sélectionné parmi :
3,4-diméthyle-2,5-diphényle-1,3,2-oxazaborolidine;
4,4-diméthyle-2-phényle-1,3,2-oxazaborolidine;
2-butyle-4,4-diméthyle-1,3,2-oxazaborolidine;
4-méthyle-2,5-diphényle-1,3,2-oxazaborolidine;
2-butyle-3,4-diméthyle-5-phényle-1,3,2-oxazaborolidine;
2-butyle-4-méthyle-5-phényle-1,3,2-oxazaborolidine;
B-hydroxy-3,4-diméthyle-2,5-diphényle-1,3,2-oxazaborolidine;
B-hydroxy-4,4-diméthyle-2-phényle-1,3,2-oxazaborolidine;
B-hydroxy-2-butyle-4,4-diméthyle-1,3,2-oxazaborolidine;
B-hydroxy-4-méthyle-2,5-diphényle-1,3,2-oxazaborolidine;
B-hydroxy-2-butyle-3,4-diméthyle-5-phényle-1,3,2-oxazaborolidine; and
B-hydroxy-2-butyle-4-méthyle-5-phényle-1,3,2-oxazaborolidine.

7. La composition selon la revendication 1 ou 4 dans laquelle n = 0, R₁est sélectionné parmi un alkyle en C₁-C₈ et un aryle; R₂ est sélectionné parmi l'hydrogène et un alkyle en C₁-C₈; R₃ et R₄ sont chacun indépendamment sélectionnés parmi l'hydrogène, un alkyle en C₁-C₈ et un aryle; R₅ et R₆ sont chacun indépendamment sélectionnés parmi l'hydrogène, un alkyle en C₁-C₈ et un aryle; et R₁' ensemble avec R₂' sont un groupe -OR₁₀-, dans lequel R₁₀ est un alkyle en C₁-C₃, formant ainsi un hétérocycle fusionné avec le cycle oxazaborolidine.

8. La composition selon la revendication 7 dans laquelle ledit composé est sélectionné parmi:
[(2-)-N,O,O'[2,2'-Iminobis[éthanolato]]]-2-n-butyle de bore; et
[(2-)-N,O,O'[2,2'-lminobis[éthanolato]]]-2-phényle de bore.

9. La composition selon la revendication 1 dans laquelle ladite modulation est une diminution dudit paramètre relié à une bactérie.

10. La composition selon la revendication 1 dans laquelle ledit paramètre relié à une bactérie est l'adhésion de la bactérie à son substrat et ladite modulation est une augmentation.

11. La composition selon la revendication 1 dans laquelle ledit paramètre relié à une bactérie est la viabilité de la bactérie, ladite modulation est une diminution et ledit composé est sélectionné parmi le 3,4-diméthyle-2,5-diphényle-1,3,2-oxazaborolidine; and 2-butyle-3,4-diméthyle-5-phényle-1,3,2-oxazaborolidine.

12. La composition selon la revendication 1 dans laquelle ledit paramètre relié à une bactérie est l'adhésion de la bactérie à son substrat, ladite modulation est une diminution, et ledit composé est sélectionné parmi le 2-butyle-4,4-diméthyle-1,3,2-oxazaborolidine, 2-butyle-3,4-diméthyle-5-phényle-1,3,2-oxazaborolidine, 2-butyle-4-méthyle-5-phényle-1,3,2-oxazaborolidine, et le [(2-)-N,O,O'[2,2'-iminobis[ethanolato]]]-2-n-butyle de bore.

13. La composition selon la revendication 1 dans laquelle ledit paramètre relié à une bactérie est l'adhésion de la bactérie à son substrat, ladite modulation est une augmentation, et ledit composé est sélectionné parmi le 3,4-diméthyle-2,5-diphényle-1,3,2-oxazaborolidine, 4,4-diméthyle-2-phényle-1,3,2-oxazaborolidine, 4-méthyle-2,5-diphényle-1,3,2-oxazaborolidine, et le [(2-)-N,O,O'[2,2'-Iminobis[éthanolato]]]-2-phényle de bore.

14. La composition selon la revendication 1 dans laquelle ledit paramètre relié à une bactérie est l'effet sur le quorum sensing, ladite modulation est une augmentation, et ledit composé est le 3,4-diméthyle-2,5-diphényle-1,3,2-oxazaborolidine.

15. La composition selon la revendication 1 dans laquelle ledit paramètre relié à une bactérie est l'effet sur le quorum sensing, ladite modulation est une diminution, et ledit composé est le 4,4-diméthyle-2-phényle-1,3,2-oxazaborolidine.

16. Un composé de formule I incluant un sel pharmaceutiquement acceptable, le solvate, l'hydrate, les isomères, et stéréo-isomères de celui-ci: 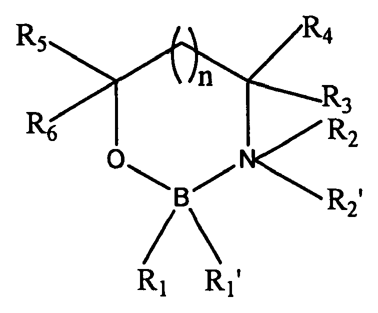 dans lequel,
n = 0;
R₁ est sélectionné parmi un alkyle en C₁-C₈ et un aryle;
R₂ est sélectionné parmi l'hydrogène et un alkyle en C₁-C₈;
R₃ et R₄ sont chacun indépendamment sélectionnés parmi l'hydrogène, un alkyle en C₁-C₈ et un aryle;
R₅ et R₆ sont chacun indépendamment sélectionnés parmi l'hydrogène, un alkyle en C₁-C₈ et un aryle; et
R₁' ensemble avec R₂' sont un groupe -OR₁₀-, dans lequel R₁₀ est un alkyle en C₁-C₃, formant ainsi un hétérocycle fusionné avec le cycle oxazaborolidine;
avec la condition que les composés suivants sont exclus, dans lesquels:
R₁ est un groupe alkyle en C₂-C₄ ou C₆, R₁' ensemble avec R₂' sont un groupe -OR₁₀-, dans lequel R₁₀ est un groupe alkyle en C₂, et R₂ à R₆ représentent un atome d'hydrogène; et
R₁ est un groupe alkyle en C₆, R₁' ensemble avec R₂' sont un groupe -OR₁₀-, dans lequel R₁₀ est un groupe alkyle en C₂, R₂ est un groupe alkyle en C₁ et R₃ à R₆ représentent un atome d'hydrogène.

17. Le composé de la revendication 16 dans lequel ledit R₁₀ est l'éthyle.

18. Le composé de la revendication 16 dans lequel ledit R₁ est l'aryle.

19. Le composé de la revendication 16 dans lequel ledit aryle de R₁ est un phényle.

20. Le composé de la revendication 16 dans lequel ledit alkyle en C₁-C₈ est un alkyle en C₁-C₄.

21. Le composé de la revendication 16 dans lequel ledit R₁ est un alkyle en C₁-C₈.

22. Le composé de la revendication 16 dans lequel ledit R₁ est un alkyle en C₁-C₄.

23. Le composé de la revendication 16 dans lequel ledit alkyle en C₁-C₈ de R₁ est un butyle.

24. Le composé de la revendication 16 dans lequel ledit R₂ est l'hydrogène.

25. Le composé de la revendication 16 dans lequel ledit R₂ est un alkyle en C₁-C₈.

26. Le composé de la revendication 16 dans lequel ledit alkyle en C₁-C₈ de R₂ est le méthyle.

27. Le composé de la revendication 16 dans lequel R₃, R₄, R₅ et R₆ sont l'hydrogène.

28. Le composé de la revendication 16 étant le [(2-)-N,O,O'[2,2'-iminobis[éthanolato]]]-2-phényle de bore.

29. Une composition pour diminuer la croissance bactérienne comprenant un composé de formule (I) comme défini dans une revendication quelconque parmi 1-28.

30. Une composition pour diminuer la susceptibilité d'une bactérie aux effets cytotoxiques d'autres agents antibactériens comprenant le composé de formule (I) comme défini dans une revendication quelconque parmi 1-28.

31. Une composition pharmaceutique comprenant un excipient pharmaceutiquement acceptable, et en tant que principe actif le composé de formule I comme défini dans une revendication quelconque parmi 1-28.

32. La composition pharmaceutique selon la revendication 31 dans laquelle ladite composition est pour le traitement, la prévention, ou l'amélioration d'une infection bactérienne.

33. La composition pharmaceutique selon la revendication 31 dans laquelle ladite composition est pour l'augmentation de la susceptibilité d'une bactérie aux effets cytotoxiques d'autres agents antibactériens.

34. La composition pharmaceutique selon la revendication 31 comprenant de plus au moins un autre agent antibactérien.

35. Utilisation des composés de formule I comme définis dans une revendication quelconque parmi 1-28 pour la préparation d'une préparation pharmaceutique pour diminuer la croissance bactérienne.
